# EUROPEAN PATENT APPLICATION

(11) **EP 2 716 655 A1**
(43) Date of publication of application: **09.04.2014**
(21) Application number: 12187318.6
(22) Date of filing: 04.10.2012
(51) Int. Cl.: C07K 16/10, G01N 33/58

(54) **Neutralizing antibodies directed against Hepatitis C virus ectodomain glycoprotein E2**

(71) Applicant: INSTITUT PASTEUR, 75015 Paris (FR)
(72) Inventor: Krey, Thomas, 78190 Trappes (FR); Tarr, Alexander W., Beeston Nottinghamshire NG91GJ (GB); Lafaye, Pierre, 92240 Malakoff (FR); Ball, Jonathan K., Newark Nottinghamshire NG22 9DG (GB); Rey, Felix A., 91190 Gif-sur-Yvette (FR); Jestin, Jean-Luc, 75003 Paris (FR)
(74) Representative: Rançon, Xavier Lucien Abel

(57) **Abstract**

The present invention relates to antibodies or fragments thereof, such as variable domains of camelid heavy-chain antibodies, directed against Hepatitis C virus glycoprotein E2, and uses thereof for preparing therapeutic or diagnostic agents.

## Description

The present invention relates to neutralizing antibodies and fragments thereof directed against the major Hepatitis C virus glycoprotein E2, and their use for treating or diagnosing Hepatitis C virus infection. Particularly, these antibody fragments are variable domains from camelid heavy-chain antibodies (VHH).

An estimated 180 million people worldwide are infected with Hepatitis C virus (HCV). Immune control of infection is uncommon and chronic infection occurs in approximately 80% of cases. The resulting liver disease has become the leading indication for liver transplantation. The engrafted liver is rapidly re-infected, with poor survival rates post-transplantation (Verna and Brown, 2008). HCV replication is rapid and error-prone; together these properties result in the potential for great genetic diversity. As a consequence, strains are classified into six major genotypes each differing by up to 30% of their nucleotide sequence (Simmonds *et al.,* 2005). Within an infected host the virus exists as a population of circulating variants, providing the capacity to adapt to anti-viral selection pressures such as direct acting therapies and host immunity. This has significant implications for the design of therapeutics and vaccines.

Since 1999 a combination of pegylated alpha interferon (IFN-α) and ribavirin has been the 'gold standard' therapy for chronic HCV infection. However, the limitations of this combination therapy are the associated severe side effects and, dependent on the viral genotype, variable sustained virological response (SVR) rates. While the recently introduced HCV NS3 protease inhibitors Boceprevir and Telaprevir show improved efficiency in combination with IFN-α and ribavirin for genotype 1 infections (Kwo *et al.,* 2010; McHutchison *et al.,* 2010), the side effects of these new drugs still present a major limitation to therapy (Sarrazin *et al.,* 2012) . The natural emergence of viruses resistant to both drugs (Welsch *et al.,* 2012) suggests that HCV will remain a major global health burden despite the introduction of these direct-acting antivirals.

Several studies have investigated the role of neutralizing antibodies in the course of HCV *infection in vivo.* Screening of HCV-infected patients receiving Hepatitis B polyclonal immunoglubulins containing anti-HCV antibodies suggested a protective role for anti-HCV antibodies (Feray *et al.,* 1998). Antibodies targeting the major envelope glycoprotein E2 were shown to prevent non-homologous virus infection after vaccination in chimpanzees (Youn *et al.,* 2005). Broadly neutralizing human monoclonal antibodies protected against heterologous virus challenge in a human liver-chimeric mouse model (Law *et al.,* 2007). Recent studies provided evidence that viral clearance during acute HCV infection is associated with the presence of high titers of neutralizing antibodies (Dowd *et al.,* 2009; Pestka *et al.,* 2007), directed to specific epitopes (Ndongo *et al.,* 2010). Immunotherapy with neutralizing conventional antibodies is an attractive component of a combination therapy, and may have specific application in preventing infection of naïve liver transplants. However, production of human monoclonal antibodies is expensive (Farid *et al.,* 2007), and recent reports have demonstrated that they are unable to prevent transmission of HCV directly from cell to cell (Brimacombe *et al.,* 2011; Timpe *et al.,* 2008).

It appears from the foregoing that the need for accurate antibodies for diagnosing or treating HCV infection is important.

Conventional immunoglobulins are heterotetramers composed of two heavy and two light chains with a combined molecular weight of about 150 KD. In members of the family *Camelidae* a significant proportion of serum antibodies are homodimeric IgGs with a molecular weight of about 80 kD (Hamers-Casterman *et al*., 1993). These heavy chain immunoglobulins (Ig) contain three domains and their variable region is referred to as VHH. Recombinant VHHs (~12-14 kD in size) constitute intact antigen-binding domains and exhibit a broad antigen-binding repertoire. Their hypervariable regions are expanded and exhibit unique characteristics, such as the substitution of three to four hydrophobic framework residues (which interact with the V_{L} in conventional antibodies) by more hydrophilic amino acids. To stabilize the enlarged CDRs, VHHs often possess an additional disulfide bound between CDR1 and CDR3 in dromedaries and CDR2 and CDR3 in llamas (Harmsen and De Haard, 2007; Muyldermans, 2001). The extended CDR3 loop can adopt a convex conformation, whereas conventional paratopes are limited to concave or flat structures (Muyldermans, 2001). These features allow VHHs to recognize unique epitopes that are poorly immunogenic for conventional antibodies (Lafaye *et al*., 2009; Wernery, 2001).

VHHs have been raised to target numerous viruses (reviewed in Vanlandschoot *et al*., 2011), among those HIV (McCoy *et al*., 2012; Forsman *et al*., 2008), Influenza A (Hultberg *et al.,* 2011), Poliovirus (Thys *et al*., 2010), Foot and Mouth Disease Virus (Harmsen *et al.,* 2007) and Rotavirus (van der Vaart *et al.,* 2006). To the knowledge of the inventors, there is no VHH directed against HCV.

Although VHHs are by definition monovalent antibodies, which by default exclude any avidity effect, their biological activity measured as IC₅₀ *in vitro* can be similar to conventional, bivalent antibody molecules (Thys *et al.,* 2010). Notably, for numerous virus systems an improvement of the biological activity by up to 500-fold has been observed upon di- or trimerization of a neutralizing VHH, either by fusing two identical VHHs (monospecific) or fusing two different neutralizing VHHs (bispecific) (reviewed in Vanlandschoot *et al.,* 2011).

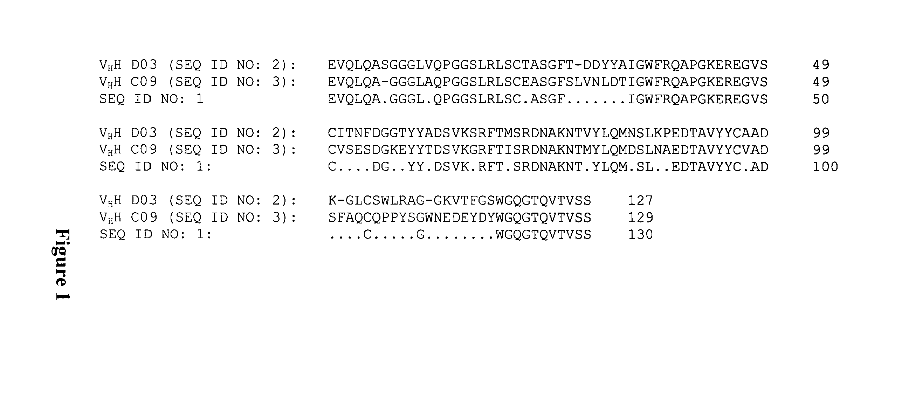

Within the framework of research that has led to the present invention, the inventors have prepared camelid heavy-chain antibodies raised by immunization with a monomeric form of the major hepatitis C virus glycoprotein E2. The initial challenge for generating broadly neutralizing VHH was the choice of a suitable immunogen. Guinea pig serum resulting from immunization with a soluble E2 ectodomain was previously demonstrated to induce antibodies that neutralize cell-cultured HCV and HCV pseudoparticles (Stamataki *et al*., 2007). Antibodies that have been induced by a glycoprotein-based vaccine (HCV genotype 1a E1/E2/p7; Choo *et al.,* 1994) neutralize isolates from genotypes 1, 4, 5 and 6, however, those antibodies failed to potently cross-neutralize isolates representing all 6 HCV genotypes (Meunier *et al.,* 2011). The inventors have used a glycoprotein construct secreting the soluble E2 ectodomain (E2e) truncated at K₇₁₅ from a genotype 2b-HCV infected patient (isolate UKN2b_2.8) (amino acid numbering is in reference to the amino acid sequence of the polyprotein of Hepatitis C virus strain H77 genotype 1a, accession number GI:130461 in the GENBANK database, and referred herein to as SEQ ID NO: 7 into *Drosophila* S2 cell supernatant. The inventors have immunized an alpaca with the E2e construct lacking the hypervariable region 1 (HVR1), in order to further increase exposure of the CD81 binding site. Correct conformation of this immunogen was demonstrated by binding to CD81 and multiple conformation-sensitive antibodies, providing evidence that this protein was similarly folded as the glycoprotein E2 in the virus particle. The inventors have thus identified and characterized four alpaca VHHs directed to different epitopes on the major Hepatitis C virus glycoprotein E2, namely VHH D03, VHH C09, VHH B11 and VHH D04. The inventors have shown that two of those, namely VHH D03 and VHH C09, have neutralizing activity. The amino acid sequence alignment between VHH D03 (represented as SEQ ID NO: 2) and VHH C09 (represented as SEQ ID NO: 3) is shown in Figure 1. The consensus sequence is represented as SEQ ID NO: 1. VHH B11 and VHH D04 do not have said consensus sequence.

An expression vector encoding the VHH D03 is contained in a bacterial strain deposited at the CNCM, 25 rue du Docteur Roux, 75724 Paris Cedex 15 on October 2, 2012, under the number I-4678. An expression vector encoding the VHH C09 is contained in a bacterial strain deposited at the CNCM, 25 rue du Docteur Roux, 75724 Paris Cedex 15 on October 2, 2012, under the number I-4679.

In particular, the monovalent VHH D03 potently neutralized a broad range of HCV primary isolates. The inventors have further demonstrated that VHH D03 neutralizes the majority of tested genotypes with an overall efficiency similar to that of the referenced bivalent, potently and broadly neutralizing human antibody 1:7 (Allander *et al.,* 2000; Johansson *et al.,* 2007). The inventors have also shown that VHH D03 inhibits cell-to-cell transmission, a route of infection that is resistant to most broadly neutralizing antibodies (Brimacombe *et al.,* 2011) and was proposed to represent a crucial *in vivo* transmission route for HCV. Furthermore, the inventors have demonstrated that the epitope recognized by this VHH D03 is distinct from any previously described for any monoclonal antibody. This epitope comprises the contacting amino acid residues G523 and T526, but does not comprise the contacting amino acid residues P525, W529, G530, D535 and N540 of a Hepatitis C virus glycoprotein E2 and is capable of binding the VHH D03. Indeed well-known mAbs 1:7 and A8 (Johansson *et al.,* 2007) bind to residues at positions G523, W529, G530 and D535, while mAb AR3A (Law *et al.,* 2008) has important contacts at S424, G523, P525, G530, D535 and N540.

Accordingly, the present invention provides an isolated neutralizing antibody or fragment thereof directed against the ectodomain of a Hepatitis C virus glycoprotein E2, characterized in that said antibody and fragment thereof bind the contacting amino acid residues G523 and T526 of said Hepatitis C virus glycoprotein E2, in reference to the amino acid sequence of the polyprotein of the Hepatitis C virus strain H77 genotype 1a of SEQ ID NO: 7.

Advantageously, said neutralizing antibody or neutralizing fragment thereof according to the present invention is a broadly neutralizing antibody or neutralizing fragment thereof that inhibits cell-to-cell transmission of HCV. A method for determining whether a neutralizing antibody or neutralizing fragment thereof is a broadly neutralizing VHH that inhibits cell-to-cell transmission of HCV is described in the Examples below.

In an embodiment, said neutralizing antibody and neutralizing fragment thereof further bind the contacting amino acid residues N415 of said Hepatitis C virus glycoprotein E2, in reference to the amino acid sequence of the polyprotein of the Hepatitis C virus strain H77 genotype 1a of SEQ ID NO: 7.

Advantageously, said neutralizing antibody and neutralizing fragment thereof do not bind amino acid residues P525, W529, G530, D535 and N540 of said Hepatitis C virus glycoprotein E2, in reference to the amino acid sequence of the polyprotein of the Hepatitis C virus strain H77 genotype 1a of SEQ ID NO: 7.

The Hepatitis C virus glycoproteins E2 and the ectodomain thereof are well known in the art. By way of example, they are described by Krey *et al.,* 2010; Lavillette *et al.,* 2005; Kato *et al*., 2001; Kolykhalov *et al.,* 1997 and Inchauspe *et al.,* 1991.

By way of example, the amino acid sequence of the ectodomain of the Hepatitis C virus glycoprotein E2 of HCV strain H77 genotype 1a and HCV strain genotype 2b isolate UKN2b_2.8 are respectively referred herein to as SEQ ID NO: 8 and 9.

The characterization of the binding of an antibody or antibody fragment according to the present invention to contacting amino acid residues of Hepatitis C virus glycoprotein E2 can be performed by substituting an amino acid residue in the ectodomain of a Hepatitis C virus glycoprotein E2 (*e.g*., with an alanine if said amino acid residue in not an alanine) and then carrying out a binding assay (see the Examples below).

The invention encompasses natural, recombinant or synthetic polyclonal or monoclonal antibodies, chimeric antibodies such as humanized antibodies, and also fragments thereof (for example: Fab, Fv, scFv) which have retained their ability to bind the ectodomain of a Hepatitis C virus glycoprotein E2 as defined above.

As used herein, the term "recombinant" refers to the use of genetic engineering methods (cloning, amplification) to produce said antibody or antibody fragment.

As used herein, the term "synthetic" refers to the production of said antibody or antibody fragment by *in vitro* chemical or enzymatic synthesis.

In a preferred embodiment, said antibody fragment is a variable antigen-binding domain of a camelid heavy-chain antibody (VHH).

As used herein, the term "VHH of camelid" (camel, dromedary, llama, alpaca, etc.) refers to the variable antigen-binding domain from a camelid heavy-chain antibody (See Nguyen *et al.,* 2000; Muyldermans, 2001 and for review Vanlandschoot *et al.,* 2011). A VHH can also be named Nanobody (Nb).

In another preferred embodiment, the VHH according to the present invention is from an alpaca (*Llama pacos*) heavy-chain antibody.

In another preferred embodiment, the antibody or antibody fragment, preferably the VHH, according to the present invention comprises or consists of the amino acid sequence SEQ ID NO: 1.

Advantageously, the VHH according to the present invention contains two disulfide bonds, one connecting CDR1 and CDR3 region of the VHH and the other connecting the framework upstream of CDR2 and the CDR3 region of the VHH.

In another preferred embodiment, the VHH according to the present invention consists of the amino acid sequence SEQ ID NO: 2 (VHH D03).

In another preferred embodiment, the antibody or fragment thereof, preferably the VHH, according to the present invention comprises one CDR (Complementarity Determining Region) from VHH D03 (SEQ ID NO: 2), selected from the group consisting of SEQ ID NO: 31 (CDR1 from VHH D03), SEQ ID NO: 32 (CDR2 from VHH D03) and SEQ ID NO: 33 (CDR3 from VHH D03), preferably two CDRs from VHH D03, *i.e.,* CDR1 (SEQ ID NO: 31) and CDR2 (SEQ ID NO: 32), or CDR1 (SEQ ID NO: 31) and CDR3 (SEQ ID NO: 33), or CDR2 (SEQ ID NO: 32) and CDR3 (SEQ ID NO: 33) from VHH D03, more preferably the three CDRs from VHH D03, *i.e.,* CDR1 (SEQ ID NO: 31), CDR2 (SEQ ID NO: 32), and CDR3 (SEQ ID NO: 33).

The antibody fragment, preferably the VHH, according to the present invention can be in the form of a monomer or a homomultimer, such as a homodimer or a homotrimer, preferably a monomer.

Antibody fragments, preferably VHHs, according to the present invention can also be included in a chimeric antibody, comprising two different or identical antibody fragments, preferably VHHs, according to the present invention and a fragment Fc of a human antibody.

A VHH according to the present invention is obtainable by the method comprising the steps of:
(a) immunizing a camelid, preferably a *Llama pacos,* with a Hepatitis C virus glycoprotein E2 (HCV E2) or the ectodomain of a Hepatitis C virus glycoprotein E2 (HCV E2e) or with the ectodomain of a Hepatitis C virus glycoprotein E2 lacking the hypervariable region 1 (HVR1) (HCV E2eΔHVR1; Krey *et al.,* 2010), preferably E2eΔHVR1; optionally boosting once or twice said camelid with HCV E2e or HCV E2eΔHVR1, preferably HCV E2e,
(b) isolating peripheral lymphocytes of the immunized camelid, obtaining the total RNA and synthesizing the corresponding cDNAs (methods are known in the art; for instance see LAFAYE *et al.,* 1995),
(c) constructing a library of cDNA fragments encoding VHH,
(d) transcribing the VHH-encoding cDNAs obtained in step (c) to mRNA, and
(e) expressing the VHH in an expression vector and, optionally purifying the expressed VHH.

In a preferred embodiment of said method, in step (a), the camelid is immunized at days 0, 21 and 35 with 250 µg of said HCV glycoprotein E2e or E2eΔHVR1.

In another preferred embodiment of said method, in step (a), the camelid is immunized with the E2eΔHVR1 of the HCV genotype 2b isolate UKN2B2.8 of SEQ ID NO: 30.

In a preferred embodiment of said method, in step (c), said library can be constructed by amplifying by PCR the DNA fragments encoding the VHH, and ligating the PCR products obtained into a phage vector.

The antibody and fragment directed against the ectodomain of a Hepatitis C virus glycoprotein E2 according to the present invention can be in a form of a chimeric antibody, comprising:
- one CDR, preferably at least two different CDRs, more preferably the three CDRs from VHH D03 (SEQ ID NO: 2), selected from the group consisting of SEQ ID NO: 31 (CDR1), SEQ ID NO: 32 (CDR2), SEQ ID NO: 33 (CDR3), and
- one or two different or the three CDRs from VHH C09 (SEQ ID NO: 3), selected from the group consisting of SEQ ID NO: 34 (CDR1), SEQ ID NO: 35 (CDR2) and SEQ ID NO: 36 (CDR3).

In an embodiment, said chimeric antibody comprises or consists of the VHH D03 of SEQ ID NO: 2 covalently linked to the VHH C09 of SEQ ID NO: 3.

The present invention also provides an isolated neutralizing antibody or fragment thereof directed against the ectodomain of a Hepatitis C virus glycoprotein E2, characterized in that said antibody and fragment thereof comprises one, preferably two different, more preferably the three CDRs from VHH C09 (SEQ ID NO: 3), selected from the group consisting of SEQ ID NO: 34 (CDR1), SEQ ID NO: 35 (CDR2) and SEQ ID NO: 36 (CDR3).

Advantageously, said fragment thereof is a VHH of camelid, preferably a VHH from an alpaca (*Llama pacos*) heavy-chain antibody, more preferably the VHH C09 of SEQ ID NO: 3.

The present invention also provides an antigen consisting of a HCV E2 ectodomain lacking the hypervariable region 1 (HVR1), corresponding to amino acids 412-715 of the polyprotein of said HCV in reference to HCV strain H77c of SEQ ID NO: 7.

In a preferred embodiment, said immunogen consists of the sequence SEQ ID NO: 30 (E2eΔHVR1).

The present invention also provides a HCV vaccine composition comprising a therapeutically effective amount of an antigen according to the present invention.

The present invention also provides the use of an antigen according to the present invention for immunizing an animal, preferably a mammal, more preferably a camelid, such as a *Llama pacos.*

The present invention also provides an isolated polypeptide comprising an antibody fragment according to the present invention, preferably a VHH or a fragment thereof, preferably at least one CRD thereof, provided that said antibody fragment comprised in said polypeptide is able to bind the ectodomain of a Hepatitis C virus glycoprotein E2 as defined above.

The present invention also provides an isolated camelid serum, preferably an alpaca serum, comprising a VHH or a polypeptide according to the present invention.

The present invention also provides an isolated polynucleotide encoding an antibody fragment, preferably a VHH or a fragment thereof, preferably at least one CRD thereof, or a polypeptide according to the present invention.

Polynucleotides according to the present invention may be obtained by well-known methods of recombinant DNA technology and/or of chemical DNA synthesis.

The present invention also provides recombinant expression cassettes comprising a polynucleotide according to the present invention under the control of a transcriptional promoter allowing the regulation of the transcription of said polynucleotide in a host cell. Said polynucleotide can also be linked to appropriate control sequences allowing the regulation of its translation in a host cell.

The present invention also provides a recombinant vector comprising a polynucleotide according to the present invention. Particularly, the recombinant vector is a recombinant expression vector encoding an antibody fragment, preferably a VHH, or a polypeptide comprising an antibody fragment according to the present invention. Advantageously, said recombinant expression vector comprises an expression cassette according to the present invention.

The present invention also provides a host cell containing a recombinant expression cassette or a recombinant vector according to the present invention. The host cell is either a prokaryotic or eukaryotic host cell.

The present invention also provides a pharmaceutical composition comprising an antibody, an antibody fragment, a polypeptide comprising an antibody fragment or a polynucleotide (such as a recombinant expression vector encoding a VHH), preferably a VHH, according to the present invention, and a pharmaceutically acceptable carrier.

When the antibody or fragment thereof, preferably the VHH, according to the present invention is administered to a human subject, then it can be humanized in order to reduce immunogenicity in human. Methods for producing humanized antibodies or fragments thereof are known in the art (Vinckle e*t al.,* 2009).

Further, the bioavailability of the antibody or antibody fragment according to the present invention can be improved by conjugating the neutralizing molecule(s) to inert carriers like albumin (Coppieters *et al,* 2006) or immunoglobulins (Harmsen *et al.,* 2005).

Said vector can be delivered to a subject by gene therapy.

As used herein, "pharmaceutically acceptable carrier" is intended to include any and all solvents, dispersion media, coatings, antibacterial and antifungal agents, isotonic and absorption delaying agents, and the like, compatible with pharmaceutical administration. Suitable carriers are described in the most recent edition of Remington's Pharmaceutical Sciences, a standard reference text in the field. Preferred examples of such carriers or diluents include, but are not limited to, water, saline, Ringer's solutions, dextrose solution, and 5% human serum albumin. Liposomes, cationic lipids and non-aqueous vehicles such as fixed oils may also be used. The use of such media and agents for pharmaceutically active substances is well known in the art. Except insofar as any conventional media or agent is incompatible with a therapeutic agent as defined hereabove, use thereof in the composition of the present invention is contemplated.

Advantageously, said pharmaceutical composition is intended for preventing or treating a Hepatitis C virus infection.

The present invention also provides an antibody, an antibody fragment, a polypeptide comprising an antibody fragment or a polynucleotide (such as a recombinant expression vector encoding a VHH), preferably a VHH, according to the present invention, for use as a medicament, preferably for preventing or treating a Hepatitis C virus infection.

The present invention also provides a method for preventing or treating a Hepatitis C virus infection, comprising administering to a subject in need thereof an antibody, an antibody fragment, a polypeptide comprising an antibody fragment or a polynucleotide encoding a VHH (such as a recombinant expression vector encoding a VHH), preferably a VHH, according to the present invention, in an amount effective to inhibit HCV infection of susceptible cells so as to thereby prevent or treat the infection.

The antibody, antibody fragment (preferably a VHH), polypeptide comprising an antibody fragment and polynucleotide according to the present invention can be orally administered to a subject (a mammal, and preferably a human). They can also be administered to said subject by injection, such as intravenous, intraperitoneal, intramuscular or subcutaneous injection.

The term "treating" includes the administration of an antibody, antibody fragment (preferably a VHH), a polypeptide comprising an antibody fragment or a polynucleotide according to the present invention to a patient who has a Hepatitis C virus infection or a symptom of Hepatitis C virus infection, with the purpose to cure, heal, alleviate, relieve, alter, remedy, ameliorate, improve, or affect the Hepatitis C virus infection, the symptoms of the Hepatitis C virus infection.

The term "preventing" means that the progression of a Hepatitis C virus infection is reduced and/or eliminated, or that the onset of a Hepatitis C virus infection is delayed or eliminated.

The present invention also provides a diagnostic agent comprising an antibody, antibody fragment, or a polypeptide comprising an antibody fragment, preferably a VHH, according to the present invention.

In an embodiment of said diagnostic agent, the antibody, antibody fragment or polypeptide comprising an antibody fragment, preferably a VHH, according to the present invention is linked, directly or indirectly, covalently or non-covalently to a detectable marker.

The detectable marker can be directly and covalently linked to the antibody, antibody fragment or polypeptide comprising an antibody fragment, preferably a VHH, according to the present invention, either to one of the terminal ends (N or C terminus) of said antibody, antibody fragment or polypeptide, or to the side chain of one of the amino acids of said antibody, antibody fragment or polypeptide. The detectable marker can also be indirectly and covalently linked to said antibody, antibody fragment or polypeptide comprising an antibody fragment through a connecting arm (*i.e*., a cross-linking reagent) either to one of the terminal ends of said antibody, antibody fragment or polypeptide, or to a side chain of one of the amino acids of said antibody, antibody fragment or polypeptide. Linking methods of a compound of interest to a peptide or antibody are well-known in the art.

In a preferred embodiment of said diagnostic agent, said detectable marker is selected from the group consisting of:
- enzymes such as horseradish peroxidase, alkaline phosphatase, glucose-6-phosphatase or beta-galactosidase;
- fluorophores such as green fluorescent protein (GFP), blue fluorescent dyes excited at wavelengths in the ultraviolet (UV) part of the spectrum (*e.g*. AMCA (7-amino-4-methylcoumarin-3-acetic acid); Alexa Fluor 350), green fluorescent dyes excited by blue light

*(e.g.* FITC, Cy2, Alexa Fluor 488), red fluorescent dyes excited by green light *(e.g.* rhodamines, Texas Red, Cy3, Alexa Fluor dyes 546, 564 and 594), or dyes excited with farred light (*e.g.* Cy5) to be visualized with electronic detectors (CCD cameras, photomultipliers);
- heavy metal chelates such as europium, lanthanum or yttrium;
- radioisotopes such as [¹⁸F]fluorodeoxyglucose, ¹¹C-, ¹²⁵I-, ¹³¹I-, ³H-, ¹⁴C-, ³⁵S, or ⁹⁹Tc- labelled compounds.

The present invention also provides a kit for diagnosing or monitoring, in a subject, a Hepatitis C virus infection, comprising an antibody, antibody fragment, preferably a VHH, a polypeptide comprising an antibody fragment or a diagnostic agent according to the present invention and an appropriate diagnostic reagent.

The appropriate diagnostic reagent is necessary for performing an assay for diagnosing or monitoring, in a subject, a Hepatitis C virus infection. The appropriate diagnostic reagent can be a solvent, a buffer, a dye, an anticoagulant, a Hepatitis C virus glycoprotein E2, the ectodomain of a Hepatitis C virus glycoprotein E2 (HCV E2e) or the ectodomain of a Hepatitis C virus glycoprotein E2 lacking the HVR1 domain.

The present invention also provides the use of an antibody, antibody fragment, preferably a VHH, a polypeptide comprising an antibody fragment or a diagnostic agent according to the present invention for diagnosing or monitoring a Hepatitis C virus infection in a subject.

The present invention also provides an *in vitro* method for diagnosing a Hepatitis C virus infection in a subject, comprising the steps of:
a) contacting *in vitro* an appropriate biological sample from said subject with an antibody, antibody fragment, preferably a VHH, a polypeptide comprising an antibody fragment or a diagnostic agent according to the present invention, and
b) determining the presence or the absence of a HCV envelope glycoprotein E2 in said biological sample,
the presence of said HCV envelope glycoprotein E2 indicating that said subject has Hepatitis C virus infection.

Step b) can be carried out by determining the presence or the absence of the antibody-antigen complex (*i.e*., antibody directed to the HCV envelope glycoprotein E2 - HCV envelope glycoprotein E2 complex).

The present invention also provides an *in vitro* method for monitoring the progression or regression of a Hepatitis C virus infection in a subject, comprising the steps of:
a) contacting *in vitro* an appropriate biological sample from said subject with an antibody, antibody fragment, preferably a VHH, a polypeptide comprising an antibody fragment or a diagnostic agent according to the present invention,
b) determining the amount of HCV envelope glycoprotein E2 in said biological sample, and
c) comparing the amount determined in step (b) with the amount of HCV envelope glycoprotein E2 previously obtained for said subject,
a significant increase in amount of HCV envelope glycoprotein E2 constituting a marker of the progression of said HCV infection and a significant decrease of HCV envelope glycoprotein E2 constituting a marker of the regression of said HCV infection.

As used herein the terms "significant increase" and "significant decrease" refer to a higher amount or lower amount respectively of HCV envelope glycoprotein E2 in an appropriate biological sample with respect to the amount of HCV envelope glycoprotein E2 in an appropriate biological sample from said subject, that was previously determined and used as a reference amount.

Step b) can be carried out by determining the presence or the absence of the antibody-antigen complex (*i.e*., antibody directed to the HCV envelope glycoprotein E2 - HCV envelope glycoprotein E2 complex).

Said appropriate biological sample can be blood, serum, urine or a liver biopsy.

In addition to the preceding features, the invention further comprises other features which will emerge from the following description, which refers to examples illustrating the present invention, as well as to the appended figures.
**Figure 1** represents the sequence alignment between the amino acid sequences of VHH D03 (SEQ IDNO: 2) and VHH C09 (SEQ ID NO: 3).

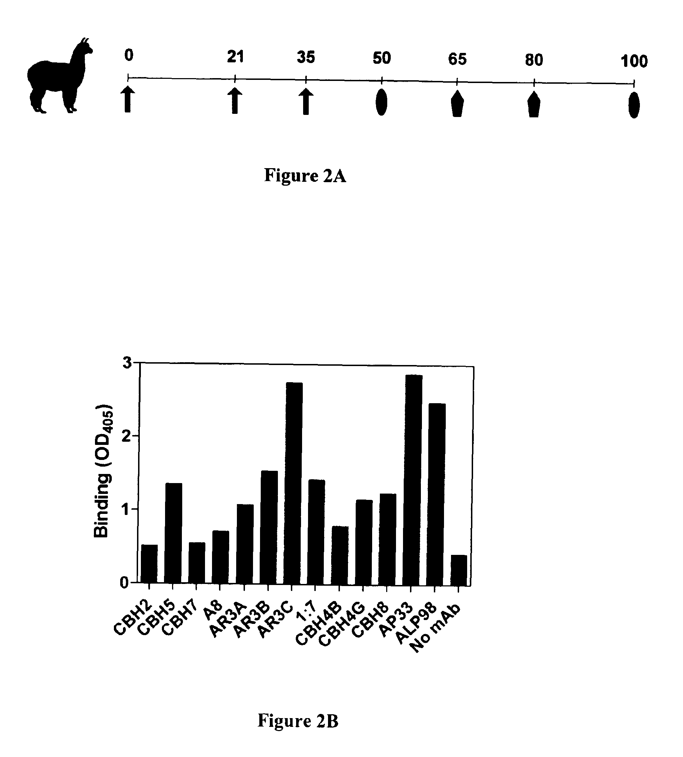

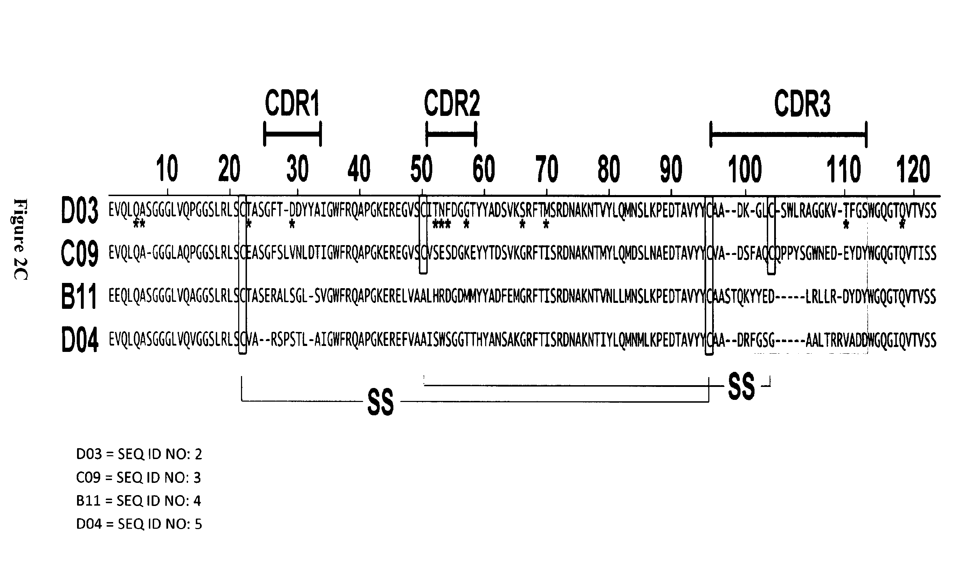

**Figure 2** represents the generation of alpaca heavy chain antibodies directed against HCV E2. (A) One alpaca was immunized and boosted with HCV E2e lacking the HVR1 domain (amino acids 412-715 of the HCV polyprotein; E2eΔHVR1) from patient isolate UKN2B2_8 (arrows). Serum samples (ellipses) were taken at day 50 to screen the humoral immune response by ELISA and at day 100 to generate a phage display library expressing VHH on the surface. In between serum sampling the animal was boosted twice with full-length ectodomain E2e (polygons) (Krey *et al.,* 2010) to increase the induced immune response. (B) The native conformation of the immunogen was evaluated by ELISA assay using human mAbs recognising discrete conformation-sensitive epitopes (CBH2, CBH5, CBH7, A8, AR3A, AR3B, AR3C, 1:7, CBH4B, CBH4G and CBH8), and two well-characterised murine mAbs recognizing linear epitopes (AP33 and ALP98) (Allander *et al.,* 2000; Hadlock *et al*., 2000; Johansson *et al.,* 2007; Law *et al.,* 2007; Owsianka *et al.,* 2005).
   The immunogen was recognized by many of the mAbs that recognize the CD81 binding region (CBH5, A8, AR3A, AR3B, AR3C, 1:7, AP33), as well others that bind to epitopes outside this region (CBH4B, CBH4G, ALP98). (C) Amino acid alignment of the four isolated VHHs. Cysteines are boxed, the disulfide connectivity is indicated below the alignment, bars show the positions of the CDRs, which are shaded, according to the IMGT nomenclature (Lefranc *et al.,* 2009). Positions of somatic mutations during maturation of VHH D03 as revealed by an alignment with the closest homologous germline sequence (IGHV1S1*01) suggested by IMGT V-QUEST and junction analysis (Lefranc *et al.,* 2009) are marked by an asterisk below the sequence.

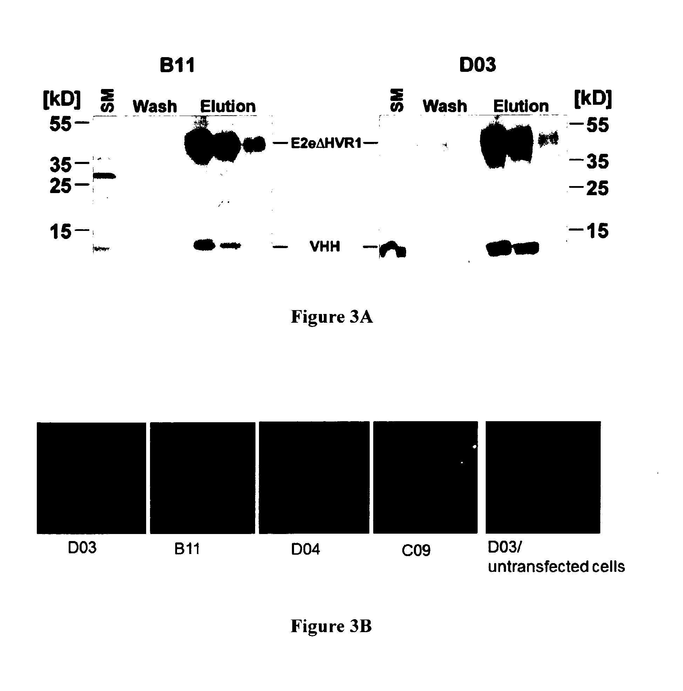

**Figure 3** represents the functional characterization of VHH. (A) E2eΔHVR1 was bound to a StrepTactin Superflow mini column and subsequently used to pull down the IMAC purified (B11, right panel) or SEC purified (D03, left panel) VHH directed against HCV E2. Eluted complexes were analyzed by SDS-PAGE under non-reducing conditions followed by Coomassie Blue staining. (B) Biotinylated VHH were bound to E1E2 proteins of strain H77 expressed on the surface of HEK293T cells transfected with an E1E2 expression construct. All four VHH were found to bind to these proteins as determined by immunofluorescent microscopy using Streptavidin-Alexafluor488 detection. Background fluorescence observed when binding each VHH to mock transfected cells was subtracted from these images. (C) CD81 binding inhibition was assayed by binding each VHH to immobilized E2e ΔHVR1 in the presence of a soluble CD81-LEL-His construct (Kitadokoro *et al.,* 2001).

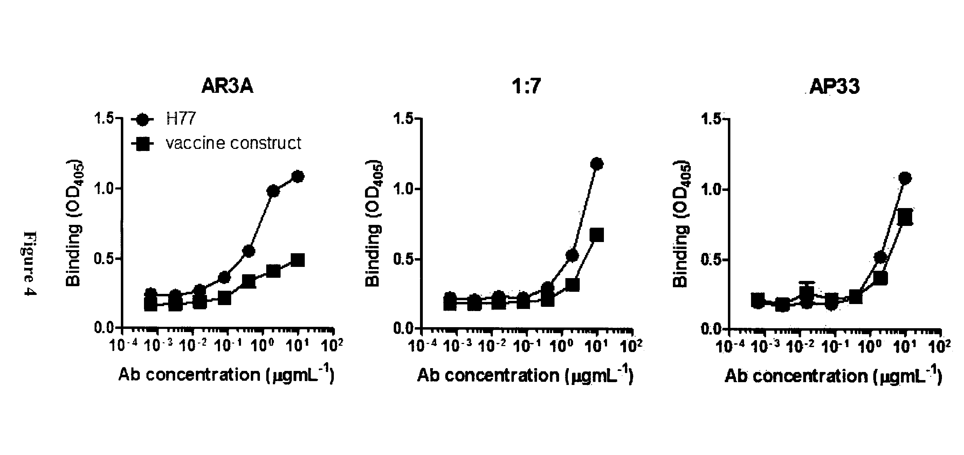

**Figure 4** represents the analysis of the native conformation of the immunogen. The overall conformation of HCV E2e lacking the HVR1 (amino acids 412-715 of the HCV polyprotein; E2eΔHVR1) used as immunogen was validated by dose-response curves for the three well-characterized monoclonal antibodies AR3A, 1:7 and AP33.

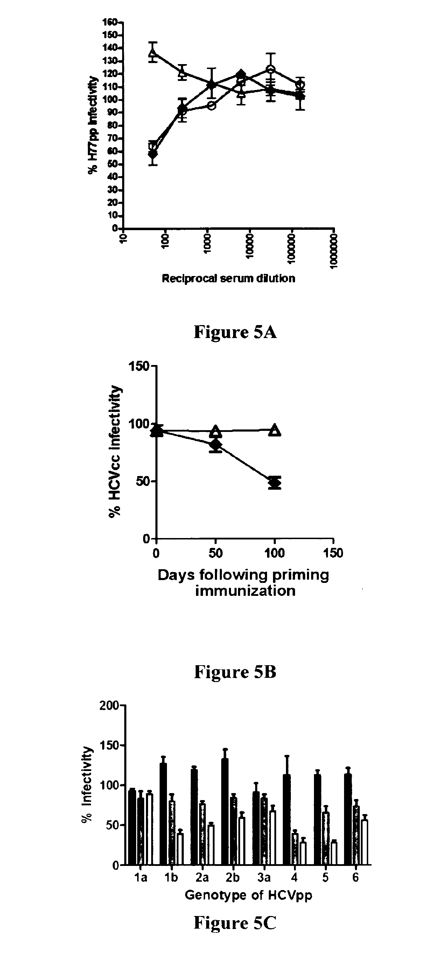

**Figure 5** represents the neutralizing activity of the alpaca serum. (A) Immune alpaca sera neutralized entry of HCVpp. Dilutions of immune sera sampled at day 50 (closed diamonds) and day 100 (open circles) were incubated with HCV pseudoparticles possessing the envelope glycoproteins of strain H77. Sera from both timepoints neutralized entry equally, while sera from an animal immunized with tetanus toxin have no neutralizing effect (open triangles). (B) Performing neutralization experiments with wild type JFH1 in cell culture revealed greater neutralization potency for the sample taken at day 100 compared to day 50. Serum at a dilution of 1/100 was incubated with 100 FFU of JFH1 before infecting Huh7.5 cells. Immune sera (closed diamonds) neutralized entry, while sera from an individual immunized with tetanus toxin (open triangles) had no neutralizing effect. (C) The breadth of neutralization was assessed utilizing a panel of HCV pseudoparticles representing genotypes 1-6. Differences in the neutralizing potency were observed between samples when incubated with serum from day 50 (grey bars) or day 100 (white bars). The sample representing genotype 1a was resistant to neutralization by serum from both timepoints but for all other patient isolates, neutralization was observed for the later sample. No inhibition of entry was observed with control serum (black bars).

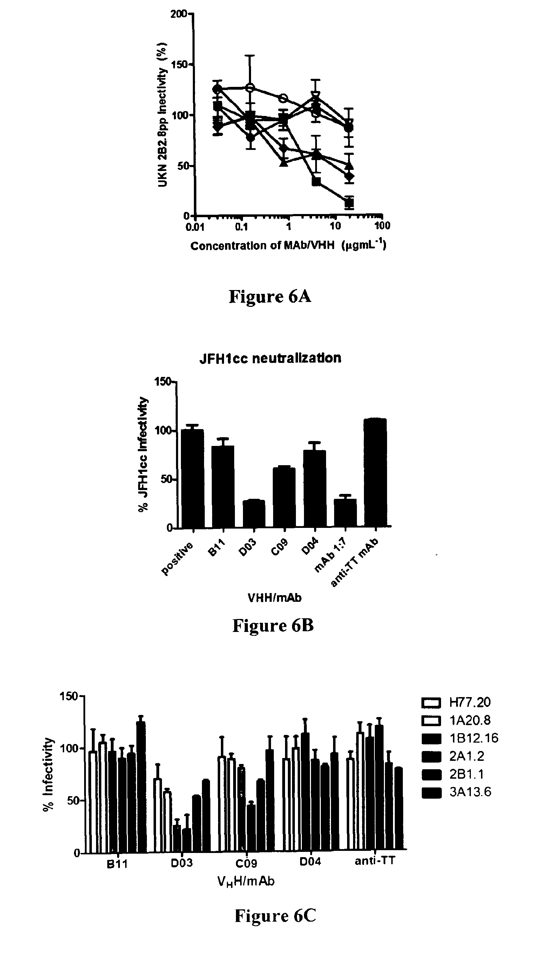

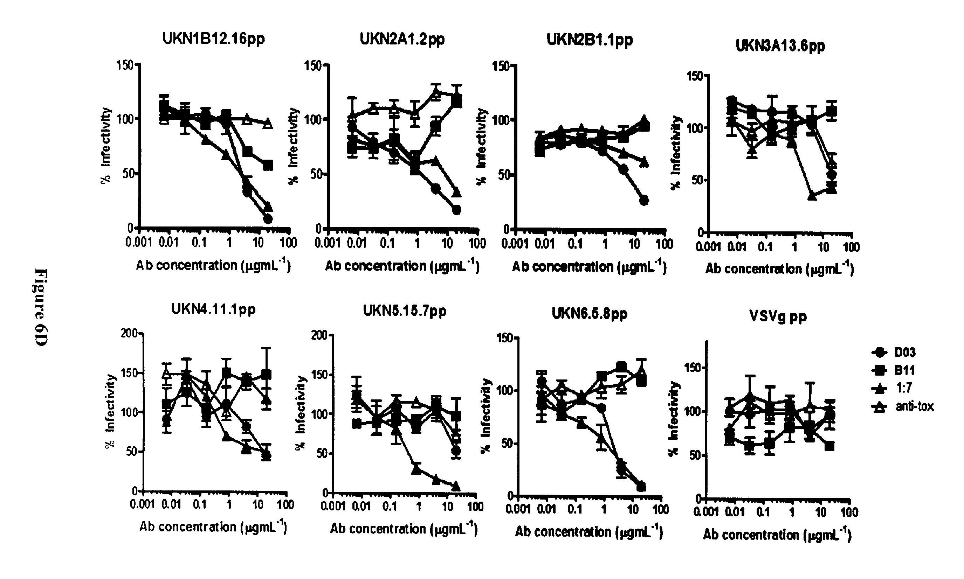

**Figure 6** represents the neutralization activity of VHH. (A) Autologous neutralization potency of each VHH was assessed using the vaccine strain UKN2B2.8. Each VHH was incubated at the indicated concentration with HCVpp bearing the UKN2B2.8 E1E2 proteins, and used to infect Huh7 cells. Dose dependent neutralization was observed for VHH D03 (closed squares) and C09, (closed triangles), while B11 (closed circles) and D04 (open triangles) had no observed neutralizing effect. D03 was by far the most potent VHH, exceeding the neutralization observed for the potently neutralizing anti-E2 mAb 1:7 (closed diamonds) serving as a control. No neutralization was observed with the control anti-tetanus toxin mAb (open circles). (B) The neutralizing potency of each isolated VHH was determined using cell-cultured JFH1 particles. Each VHH was incubated at a concentration of 10µgmL-1 with 100FFU of JFH1 particles before addition to target Huh7.5 cells. Neutralization was compared with the potently neutralizing mAb 1:7, and an irrelevant anti-tetanus toxin VHH. While VHH B11 and VHH D04 had little effect on JFH1 entry, VHH C09 showed limited inhibition and VHH D03 had much greater neutralizing potency, comparable to neutralization by mAb 1:7. (C) A neutralization screen utilizing heterologous HCV pseudoparticles representing HCV genotypes 1-3 revealed that VHH D03 possessed the greatest neutralizing activity, with VHH C09 having neutralization activity limited to genotype 2 pseudoparticles. VHHs B11 and D04 had no effect on entry of HCVpp of any genotype. Neutralizing potency of D03 was greatest for samples representing genotpyes 1b (UKN 1B12.16), 2a (UKN1A2.1) and 2b (UKN2B1.1), while 1a (H77, UKN1A20.8) and 3a (UKN3A1.28) were more resistant to neutralization. VHH samples were incubated at a concentration of 10µgmL⁻¹ with each HCVpp preparation before adding to target Huh7 cells. (D) To assess the cross-genotype efficacy of VHH D03, dose-dependent neutralization of HCV primary isolates was assessed for VHH D03 (closed circles), using VHH B11 (closed squares) and anti-tetanus toxin VHH (open triangles) as controls. Neutralization was compared to that of mAb 1:7 (closed triangles), which had previously been demonstrated to neutralize these isolates. Neutralization potency depended on the isolate used; many isolates were neutralized comparably by both VHH D03 and mAb 1:7, but UKN2A1.2 and UKN2B1.1 were more easily neutralized by VHH D03 than mAb 1:7.

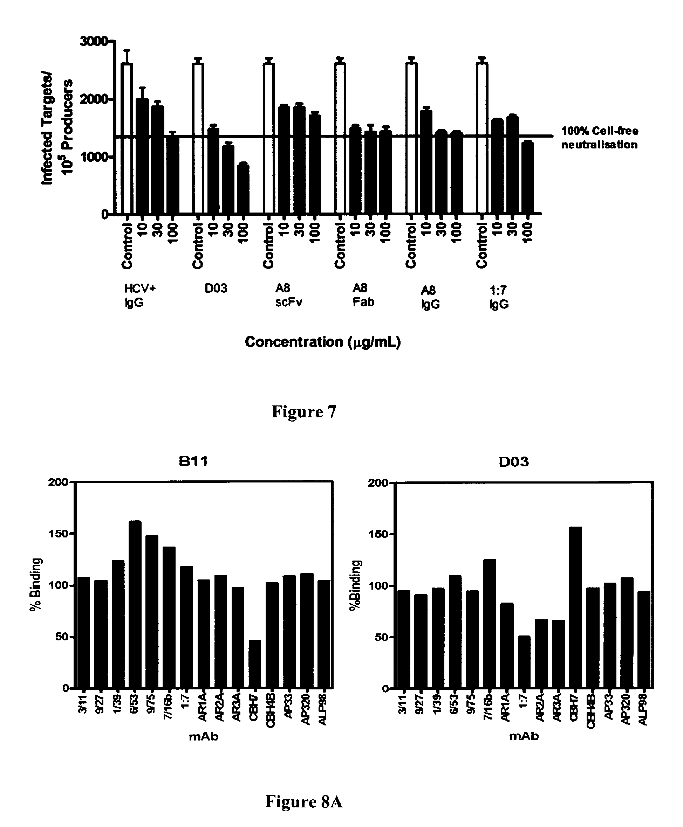

**Figure 7** represents the inhibition of cell to cell transmission by the VHH D03. Transmission between infected and naïve cells was achieved using co-cultures of CMFDA-labelled JFH-1 infected Huh7.5 cells and uninfected cells in the presence of antibodies that completely neutralize cell-free virus. Following culture, analysis by flow cytometry of CFMDA-/HCV NS5A+ cells revealed the number of direct transmission events. The presence of JFH-1 virions in cell supernatants was determined by titration; the point at which no cell free virus was detected, is highlighted by the bold line. The VHH was compared to fragments of the broadly neutralizing anti-E2 mAb A8.

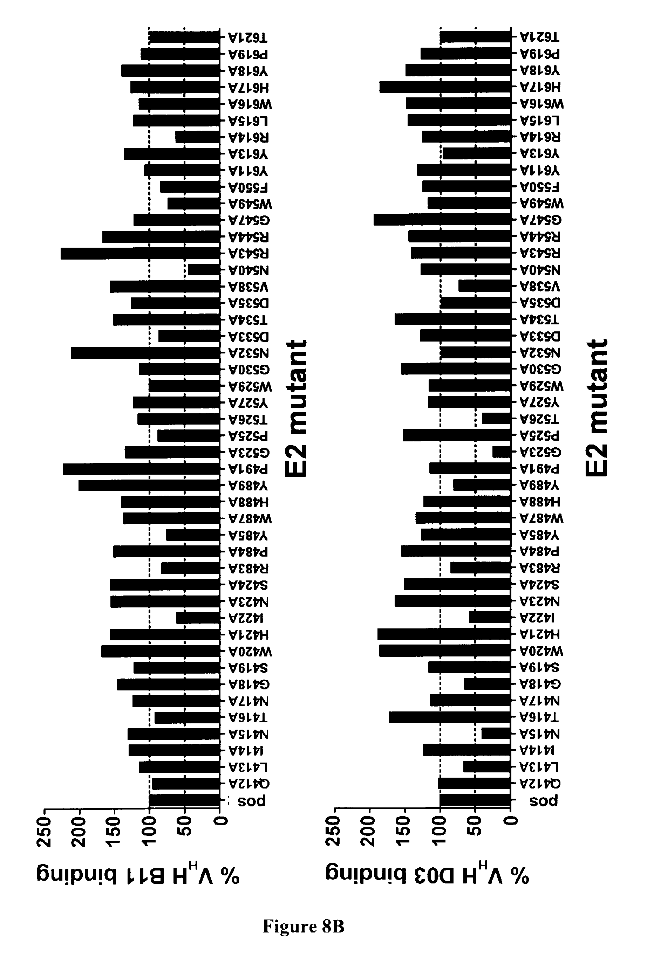

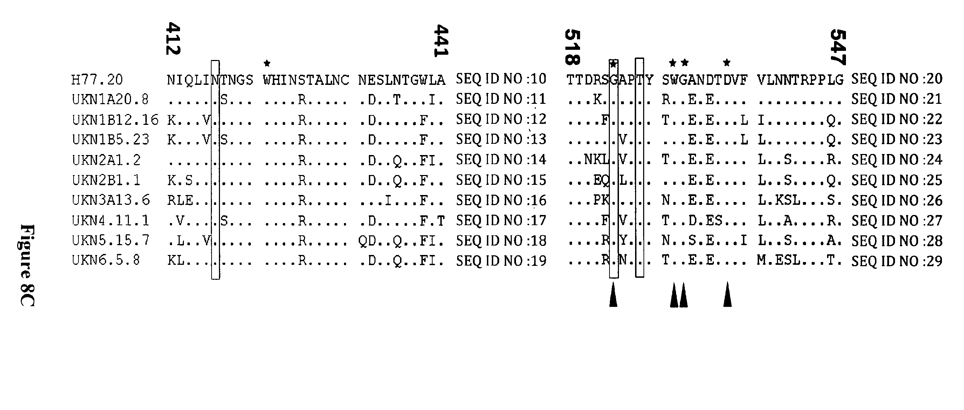

**Figure 8** represents the mapping of the epitope recognized by the VHH D03. (A) A panel of well-characterized mAbs with epitopes covering much of the surface of the E2 protein was used for competition assays with both VHH B11 and D03. Binding of a half-maximal concentration of each mAb was performed alone, or in the presence of each VHH at 10µgmL⁻¹. B11 competed only with CBH-7. In contrast, VHH D03 competed with mAbs 1:7, AR2A, and AR3A, suggesting that the epitope for this VHH overlaps the CD81 binding site. (B) Binding of VHH D03 and B11 to a panel of alanine replacement mutants was assessed by immunofluorescence on cells expressing each individual mutant. Substitution of N415, G523 and T526 of the HCV polyprotein resulted in greater than 50% reduction in binding of VHH D03, while N540A of the HCV polyprotein was the only substitution that reduced binding of B11 by greater than 50%. (C) Alignment of HCV isolates used in neutralization and binding assays. Residues identified as being important to binding of VHH D03 are boxed. Asterisks highlight residues involved in interaction with the cellular receptor CD81, and the contact residues for mAbs 1:7 and A8 are labeled by triangles.

### EXAMPLE: BROADLY NEUTRALIZING ALPACA VARIABLE HEAVY DOMAIN OF HEAVY CHAIN IgG (VHH) BLOCKS CELL-FREE AND CELL-CELL ENTRY OF HEPATITIS C VIRUS

### 1) Materials and Methods

### Antigen

As main antigen it was used the HCV E2e of the genotype 2b isolate UKN2B2.8 (Lavillette *et al.,* 2005) lacking the hypervariable region 1 (HVR1) (SEQ ID NO: 30; E2eΔHVR1) produced in *Drosophila* S2 cells as previously described (Krey *et al.,* 2010). E2eΔHVR1 (SEQ ID NO: 30) corresponds to the fragment 412-715 of the HCV genotype 2b isolate UKN2B2.8 polyprotein (amino acid numbering is in reference to the amino acid sequence of the polyprotein of Hepatitis C virus strain H77 genotype 1a, accession number GI:130461 in the GENBANK database, and referred herein to as SEQ ID NO: 7). To verify the overall conformation 25µg of E2eΔHVR1 and 30µg of either conformation-dependent Fab-fragments e137 (Perotti *et al.,* 2008) or CBH-4D (Hadlock *et al.,* 2000), respectively, were incubated as isolated proteins as well as in complex for 1h at 10°C followed by analysis on a Superdex200 Mini column (column volume 3 ml, Amersham). Size exclusion chromatography (SEC) profiles were overlaid and used to determine the elution volumes.

### Induction of a humoral immune response in an alpaca

250 µL of HCV E2eΔHVR1 (1mg/mL) produced in *Drosophila* S2 cells as previously described (Krey *et al.,* 2010) was mixed with 250 µL of Freund's complete adjuvant for the first immunization, and with 250 µL of Freund incomplete adjuvant for the following immunizations. One young adult male alpaca (*Llama pacos*) was immunized at days 0, 21 and 35 with 250 µg of the immunogen. At day 50 a serum sample was taken and the immune response was monitored by the titration of serum samples by ELISA using HCV E2e full-length ectodomain as antigen. Subsequently the alpaca was boosted twice with the homologous HCV E2e full-length ectodomain (amino acids 384-715 of the HCV polyprotein) at 15 day intervals.

### Library construction

The blood of the immunized animal was collected at day 100 and the peripheral blood lymphocytes were isolated by centrifugation on a Ficoll (Pharmacia) discontinuous gradient and stored at -80 °C until further use. Total RNA and cDNA was obtained as previously described (Lafaye *et al.,* 1995). DNA fragments encoding VHH domains were amplified by PCR using CH2FORTA4 and VHBACKA6 primers, which anneal to the 3' and 5' flanking region of the V_{H} genes, respectively. The amplified product was used as template in a second round of PCR using either the primers VHBACKA4 and VHFOR36 or the primers VHBACKA4 and L_{HH} (5' GGACTAGTTGCGGCCGCTGGTTGTGGTTTTGGTGT CTTGGG-3'; SEQ ID NO: 6) specific for the long hinge homodimeric antibody (Lafaye *et al.,* 2009). The primers were complementary to the 5' and 3' ends of the amplified product and incorporated *SfiI* and *NotI* restriction sites at the ends of the VHH genes. The PCR products were digested and ligated into phage expression vector pHEN1. The resulting library was composed of two sub-libraries, one derived from VHH DNA-encoding genes with no hinge and the other from long hinge antibody genes. Phages were produced and isolated using both sub-libraries, and subsequently pooled.

The library was panned against HCV E2e first with the antigen coated on immunotubes as previously described (Cardoso *et al.,* 2000). Nunc Immunotubes (Maxisorp) tubes were coated overnight at 4°C with the antigen (10 µg/ml) in PBS. Phages (10¹³ transducing units) were biopanned by incubation with the coated tubes for 1h at 37°C under gentle agitation.

HCV E2e contained a strep-tag at the C terminus end and in a second panning the protein was bound on Strep-Tactin coated magnetic beads (MagStrep « type 2HC » beads, IBA). The library was panned by incubation with the coated beads for 1hour at 37°C. The blocking agent was changed at every round: 2% skimmed milk, Licor diluted 1:4, and 4% BSA were respectively used. The concentration of HCV E2e bound on beads decreased at every round of panning with respectively 100 nM, 50nM and 10 nM of protein. Following panning, phage clones were screened by standard ELISA procedures using a HRP/anti-M13 monoclonal antibody conjugate (GE Healthcare) for detection.

### Expression of VHH

The coding sequence of the selected VHHs in vector pHEN1 was sub-cloned into a modified bacterial expression vector pET28a containing an 8-Histidine tag using the *NcoI* and *NotI* restriction sites. Transformed *E. coli* Rosetta-Gami2(DE3) cells expressed VHHs in the cytoplasm after induction with IPTG 0.2mM overnight at 16°C. Purified VHHs were obtained by IMAC from cytoplasmic extracts using a HiTrap crude column charged with Ni²⁺ (GE Healthcare) according to the manufacturer's instructions followed by size exclusion chromatography using a Superdex 75 column (GE Healthcare).

### Pull-down of VHHs by E2e

50µg of E2eΔHVR1 was bound to a StrepTactin Superflow mini column (column volume 0.2ml, IBA) and washed with 10 column volumes of washing buffer. Subsequently, 30µg of IMAC purified (B11) or SEC purified (D03) VHH directed against HCV E2 were added, followed by washing with 3x5 column volumes. Complexes were eluted in 3x1 column volumes elution buffer and concentrated 20-fold by ultrafiltration. The concentrated fractions were analyzed by SDS-PAGE followed by Coomassie Blue staining.

### Crystallization, data collection and structure determination

Crystals of the VHH D03 were grown at 293 K using the hanging-drop vapor-diffusion method in drops containing 1.2 µL protein (~20 mg/ml in 10 mM TRIS pH 8.0, 150 mM NaCl) mixed with 1.2 µL reservoir solution containing 2005mM LiSO4. Diffraction quality rod-like crystals appeared after two to three weeks and were flash-frozen in mother liquor containing 18% (v/v) Glycerol. Spacegroups and cell dimensions of the crystals, resolution limits, data collection details and refinement statistics are summarized in Table 1 below.
Table 1: Crystallographic data for **VHH D03** VHH D03
**Data collection**
Space group
P65
Cell dimensions
a, b, c (Å)
52.81 52.81 182.47
α, β, γ (°)
90 90 120
Resolution (A)
50.00-1.76 (1.86-1.76)
Rmerge
0.103 (0.266)
I/σI
7.7 (1.6)
Completeness (%)
94.7 (69.5)
Redundancy
5.0 (1.7)
**Refinement**
Resolution (Å)
44.36-1.76
No. reflections
26863
Rwork/ Rfree
0.182 / 0.204
**No. atoms**
Protein
1997
Ligand
-
Water
133
**B-factors**
Protein
35.55
**R.m.s. deviations**
Bond length (A)
0.01
Bond angles (°)
1.14
* Values in parentheses correspond to the highest resolution shell. R.m.s. deviation - Root-mean-square deviation.

Data were collected at the Swiss Light Source (PX I). Data were processed with XDS (Kabsch, 1988) and scaling and reduction was performed using Pointless (Evans, 2005) and programs from the CCP4 suite (Collaborative Computational Project, 1994). The crystal structure of the VHH was determined by the molecular replacement method using Phaser (McCoy *et al.,* 2007) and a VHH (PDB 3STB) as search model. Model building was performed using Coot (Emsley *et al.,* 2010) and refinement was done using AutoBuster (Bricogne *et al.,* 2010).

### Neutralization of entry of HCV pseudoparticles by immune sera and VHH

HCV pseudoparticles representing genetically diverse primary isolates were generated as previously described (Lavillette *et al.,* 2005; Tarr *et al.,* 2007; *Tarr et al.,* 2011). Briefly, HEK293T cells were co-transfected with plasmids encoding i) amino acids 170-746 of the HCV polyprotein (Genbank accession number GI:130461), ii) a murine leukemia virus (MLV) *Δenv* backbone, and iii) a luciferase reporter gene flanked by the MLV long terminal repeats (LTRs). 72 hours after transfection HCV pseudoparticles were recovered from cell supernatants, and filtered through a 0.45µm membrane. Pseudoparticles were mixed with dilutions of immune sera, defined concentrations of VHH, or monoclonal antibodies for one hour, then added to 1.5x10⁴ Huh7 cells. After incubation for 48 hours cells were lyzed with cell lysis buffer (Promega) and luciferase activity measured using luciferase substrate (Promega). Light output was measured with a BMG Fluostar Optima.

### Neutralization of HCVcc by immune sera and VHH

RNA transcripts of the JFH1 strain of HCV were produced with a MEGAscript^{®} T7 high yield transcription kit (Ambion) from linearized plasmid pJFH1. Genomic transcripts were cleaned up using an RNeasy® minikit (Qiagen). For each sample, 10µg HCV RNA was electroporated into 7 × 10⁶ Huh7.5 cells (Blight *et al.,* 2002) cells using a GenePulser Xcell™ electroporator (Bio-Rad). Media harvested after 48 hours was filtered through a 0.45 µm membrane and virus particles quantified by serially diluting cell supernatants in replicates and infecting Huh7.5 cells, Fixed cells were permeabilized with 0.5% Triton X-100 and stained with anti-NS5A mAb 9E10 (Lindenbach *et al.,* 2005) followed by HRP-conjugated Polyclonal Rabbit Anti-Mouse IgG (DakoCytomation) HRP activity was detected using the Vector® NovaRED™ (Vector) substrate kit. TCID₅₀ values were calculated using the Reed & Muench method (Reed and Muench, 1938). Neutralization assays were performed by mixing 100 focus-forming units (FFU) of virus with serum, VHH, or monoclonal antibody for one hour before adding to Huh7.5 cells. Infection was detected as described above, and percentage infection determined by comparison to the number of cells infected in the absence of inhibitors.

### Binding of VHH to a panel of Alanine-substitution E1E2 point mutants.

HEK293T cells were transfected with pcDNA3.1-based vectors possessing the E1E2 genes of the H77c molecular clone of HCV bearing individual Alanine substitution point mutants (Owsianka *et al.,* 2006). After 48 hours, transfected cells were trypsinized, harvested and seeded onto Teflon-coated microscope slides. Cells were air dried and then fixed with acetone. VHH were biotinylated using a N-hydroxysuccinamide-biotin ester (Pierce) and dialyzed into PBS using Millipore Microcon buffer exchange columns. The biotinylated VHH were then incubated with the E1E2-expressing cells. Following washing, binding was detected using a streptavidin Alexafluor488 conjugate (Invitrogen), and binding visualized using an immunofluorescence microscope. Binding was measured by integrating the total light output per field of view using a 400X magnification, subtracting the values obtained using untransfected cells as a control.

### Inhibition of cell to cell transmission of HCV

HCV-infected producer Huh-7.5 cells were 5-Chloromethylfluorescein Diacetate (CMFDA)-labeled and co-cultured with unlabeled Huh-7.5 cells in a 1:1 ratio for 2 hours to allow cell-cell contacts to form. Neutralizing antibodies were then added and the culture continued for 24h. The extracellular media was collected and tested for the presence of infectious virus by infecting naïve Huh-7.5 cells. This represented the total level of infectious extracellular virus produced during the 24h period of co-culture. Purified polyclonal anti-HCV Ig (pooled from 600 donors) neutralized >95% of infectious virus.

Co-cultures of cells were fixed and permeabilized and stained for NS5A expression. Cells were analyzed by flow cytometry to identify the number of newly infected target cells (NS5A+/CMFDA-) along with the number of infected/uninfected producers in the assay. For neutralization assays, VHH or mAbs were incubated with the co-cultured cells and the residual inhibition of cell to cell transmission compared to that achieved in the presence of pooled polyclonal anti-HCV Ig.

### Competition assay between VHH and defined monoclonal antibodies

Competition ELISAs were performed as described previously (Tarr *et al.,* 2012). Essentially, *Galanthus nivalis* agglutinin (GNA)-captured E1E2 proteins were probed with human or mouse monoclonal antibodies in the presence of increasing concentrations of the anti-E2 VHH. Bound mAb was detected with an anti-species IgG antibody conjugated to alkaline phosphatase. Binding was revealed with p-nitrophenol-phosphate substrate for 30 minutes. Competition was reported as inhibition compared to an uninhibited control.

### 2) Results

### Alpaca immunization and assessment of anti-E2 serum response

One alpaca was immunized according to the protocol shown in Figure 2a with a genotype 2b HCV (isolate UKN2B2.8) E2eΔHVR1 (SEQ ID NO: 30) produced in *Drosophila* S2 cells as described previously (Krey *et al.,* 2010). This protein lacked the hypervariable region 1 (HVR1), to allow access to more conserved neutralization epitopes that have been postulated to be shielded by the HVR1 *(Prentoe et al.,* 2011; *Bankwitz et al.,* 2010). The correct conformation of E2eΔHVR1 was confirmed by testing the binding of a panel of non-overlapping well-characterized, mostly conformation-sensitive monoclonal antibodies (Figure 2b and Figure 4).

The animal was re-boosted with HCV E2e full-length ectodomain, corresponding to amino acids 384-715 of the HCV polyprotein. Immune sera were tested for their ability to inhibit entry of HCV pseudoparticles and authentic HCV cell cultured particles. Serum sampled at day 50 and day 100 had a dose-responsive inhibitory effect on HCV entry (Figure 5). Both serum samples neutralized entry of the H77c pseudoparticles equally, but the sample taken at day 100 possessed greater neutralizing potency in the JFH1cc entry model (Figure 5b). To assess the breadth of neutralization by the immune sera, inhibition of entry of a panel of patient E1E2 isolates were assessed in the HCV pseudoparticle assay. The sample taken at day 100 consistently had greater neutralizing potency against these isolates (Figure 5c).

### Identification of specific α-HCV E2 VHHs

A VHH library was generated by PCR from lymphocytes of the immunized animal into the pHEN1 vector to allow for efficient screening by phage display, yielding a library of 4x10⁷ independent clones. After the panning selection of the VHH library on E2e-coated immunotubes, 96 clones were chosen and ELISA screening revealed that all of these clones bound to HCV E2e. 48 clones were sequenced and two independent VHHs - D03 and B11 - were identified in 60.5% and 39.5% of the clones, respectively. A second panning selection of the VHH library was performed, this time on HCV E2e bound on magnetic Streptactin beads, yielding 80.8% and 11.5% represented VHH D03 (SEQ ID NO: 2) and B11 (SEQ ID NO: 4), respectively. In addition, 2 new VHH - C09 (SEQ ID NO: 3) and D04 (SEQ ID NO: 5) - were identified. A full alignment of the amino acid sequences of all four VHHs is shown in Figure 2c. The limited number of identified VHHs suggests that the specific immune response against HCV E2e is remarkably oligoclonal, with VHH D03 binding to an immunodominant epitope.

Pull-down assays were performed to verify the interaction of the recombinant VHHs with the HCV E2 ectodomain. For this purpose the Strep-tagged ectodomain of HCV E2 produced in Drosophila S2 cells (Krey *et al.,* 2010) was bound to a streptactin column and His-tagged VHH purified either by IMAC (VHH B11, Figure 3a, left panel) or by SEC (VHH D03, Figure 3a, right panel) was added. Co-elution of the complex followed by SDS-PAGE and Coomassie staining revealed that E2e efficiently bound the VHH B11 and VHH D03 (Figure 3a). Immunofluorescence was also used to confirm binding of the four VHH to E1E2 proteins expressed on HEK 293T cells. Transiently-transfected cells were probed with biotinylated VHH, and all four VHH clones were observed to bind specifically to these cells (Figure 3b).

### Recombinant VHHs neutralize HCV entry

The four independent VHH clones were all assessed for the ability to inhibit HCV entry into cells. Firstly, HCV pseudoparticles bearing the UKN2B2.8 E1 and E2 glycoproteins were used to investigate autologous neutralization (Figure 6a). Potent, dose responsive neutralization was exhibited by VHH clone D03 (>95% at 20µg mL⁻¹). VHH C09 also possessed neutralizing activity, reaching greater than 40% inhibition at the highest concentration tested. The two other VHH, B11 and VHH D04, possessed no inhibitory effect on HCV entry. These results were reproduced using the HCVcc model of entry (Figure 6b). Wild-type JFH1 virus was incubated with each VHH, and again, clone D03 had the most potent neutralizing effect. VHH C09 only had minor inhibitory effect on JFH1 entry. Combined, these data demonstrated that the VHH D03 was able to neutralize entry of HCV strains of an autologous genotype using *in vitro* models of entry.

To assess the breadth of neutralization by these VHHs, neutralization of a panel of patient-isolated E1E2 sequences was investigated using the HCVpp entry model (Bartosch *et al.,* 2003). Initially, all four VHHs were screened at a single concentration for neutralizing activity against a panel of pseudoparticles that had previously been demonstrated to represent a spectrum of neutralization resistance (Tarr *et al.,* 2011) (Figure 6c). Only sample VHH D03 possessed significant cross-neutralizing activity, sample VHH C09 inhibiting only HCVpp representing genotype 2. VHHs B11 and D04 did not possess neutralizing activity. While the most potent activity for VHH D03 was observed with the genotype 2 isolates, entry of pseudoparticles representing all three genotypes was inhibited by this VHH. To investigate this property of VHH D03 further, dose dependent neutralization of VHH D03 was investigated using a panel of pseudoparticles representing all six HCV genotypes. VHH B11 was also included as a control, as was the positive control mAb 1:7 (directed to a conserved neutralization epitope in the CD81 binding region of E2) and a negative control mAb directed to tetanus toxin. VHH D03 neutralized entry of isolates representing all six genotypes of HCV, while VHH B11 had no inhibitory effect (Figure 6d). The neutralization potency of VHH D03 was dependent on the HCV isolate investigated. Some clones, such as UKN2A1.2 and UKN2B1.1, were more easily neutralized by VHH D03 than mAb 1:7. However some, such as UKN3A13.6 and UKN5.15.7 were more refractive to neutralization by VHH D03.

### Crystal structure of VHH D03

Sequence analysis of the four identified VHHs revealed two groups - the first one resembling the standard VHH containing only one single disulfide bond and the second one containing an additional disulfide bond connecting the CDR3 with the upstream residue of CDR2. Notably, the CD81 binding inhibition assay demonstrated that both VHHs containing a second disulfide bond interfere with CD81 binding, while the two VHHs with only one disulfide bond did not neutralize HCV infection, suggesting a possible role of this disulfide additional disulfide bond in antigen binding.

No crystal structure of a llama antibody with an additional disulfide bond between CDR3 and the framework upstream of CDR2 has been reported. The VHH D03 was crystallized to gain insights into the conformation of its potential antigen binding determinants and thus into its putative binding mode to the antigen. Diffraction quality crystals of the unliganded VHH, belonging to spacegroup P6₅, could be grown within four weeks. These crystals diffracted to 1.76Å on a synchrotron source. The crystal structure of the VHH was determined using the molecular replacement method; details and statistics of the data collection, processing and refinement are given in Table 1 above. The VHH displays the standard compact fold of an immunoglobulin domain containing nine β-strand tightly packed in two β-sheets. The three complementarity determining regions (CDR1, CDR2 and CDR3) are 8, 8 and 20 residues long (according to the IMGT nomenclature, Figure 2d), respectively, the CDR3 thus being longer than the average CDR3 from both VHH and human V_{H} regions (17 and 12 residues, respectively (Vu *et al.,* 1997)). The CDR3 folds over the part of the framework region that, in conventional antibodies, forms the V_{H}-V_{L} interface and is restrained in this conformation by a disulfide bond between Cys₅₂ directly upstream of the CDR2 and Cys₁₀₅ in the CDR3. Such inter-loop disulfide bonds have been identified in both camel and llama VHH (Vu *et al.,* 1997).

Antibody maturation in VHHs frequently includes somatic mutations that improve shape or charge complementarity of the paratope (Nguyen *et al.,* 2000). Amino acid alignment of VHH D03 with its closest homologous germline gene IGHV1S1*01 and mapping of the somatic mutations on the molecular surface of the α-HCV E2 VHH D03 revealed one, four and one somatic mutations in the CDR1, CDR2 and CDR3 (Figure 2d, labeled as asterisks), respectively.

### VHH D03 inhibits cell-cell transmission

A significant limitation to therapeutic administration of antibodies is the ability of HCV to infect naïve cells directly, without being exposed to the extracellular environment. Having demonstrated a potent neutralizing effect on entry of cell-free HCV particles into susceptible cells, the possibility of VHH inhibition of cell-cell transmission was investigated. In addition to inhibiting the cell-free virions in this assay, VHH D03 also possessed the ability to inhibit transmission of HCV from infected producer cells to naïve huh7 cells (Figure 7a). This was consistent with the ability of the VHH to neutralize cell-free entry of chimeric HCVcc (Figure 7b) and the ability of the serum to neutralize HCVpp (Figure 4).

The observed neutralizing activity of VHH D03 could be attributable to either its size, or its specificity. To discriminate between these possibilities recombinant antibody fragments of the broadly neutralizing antibody A8 (Allander *et al.,* 2000; Johansson *et al.,* 2007) was expressed in *Drosophila* S2 cells as described before (Backovic *et al.,* 2010; Gilmartin *et al.,* 2012), which possesses a similar biological activity to mAb 1:7 and binds to similar residues in E2 (Johansson *et al.,* 2007). Cell-cell transmission of JFH-1 HCVcc was neither affected by the IgG molecule of A8 (MW of ~150kD), nor by any of the recombinant fragments (Fab MW of ~50kD, single chain variable fragment (scFv), MW of ~30kD), suggesting that size is not the pivotal antibody feature that determines efficient neutralization in cell-cell transmission. Instead, this indicates that the specificity of the VHH is crucial for its efficiency to inhibit cell-cell transmission.

These antibody fragments were also tested for neutralization of cell-free HCVpp entry and directly compared to VHH D03 (Figure 8). While VHH D03 more potently neutralized clone UKN2B1.1 than mAb 1:7, when using clone H77 mAb 1:7 and its fragments were much more potently neutralizing than the VHH. In agreement with the neutralization activity on cell-cell transmission this suggests that the specificity of the VHH provides its potent neutralizing properties, rather than the small size of the protein alone.

### Binding of VHH D03 to the E2 glycoproteins identifies a novel epitope overlapping the CD81 binding site

To localize the epitope recognized by VHH D03 on the surface of E2, a panel of well-characterized monoclonal antibodies raised in mice, rats or humans was assessed for direct competition for binding of VHH D03 and VHH B11 to HCV E1E2 proteins (Figure 8a). VHH D03 competed with mAbs AR3A, 1:7 and AR2A for binding to the E1E2 proteins, providing evidence that this VHH is likely to recognize an epitope overlapping the CD81 binding site in the proposed Domain 1 of E2. For comparison, competition assays were also performed with VHH B11. In contrast, binding of mAbs AR3A, 1:7 and AR2A (Law *et al.,* 2007, Johansson *et al.,* 2007) was not competed by VHH B11, but competition was observed with mAb CBH7, which recognizes a distinct epitope. To further define the epitopes of these VHH, binding of VHH D03 and VHH B 11 to a panel of alanine-substitution mutants was also performed (Figure 8b). Given that mAbs recognizing the CD81 binding site on E2 were competed by VHH D03, individual conserved residues observed in the functional patient isolates between amino acids 412 and 550 were mutated to alanine. Distinct patterns of reactivity were observed for the two VHHs. This provides further evidence that the neutralizing epitope recognized by D03 is distinct to the epitope recognized by VHH B11. Binding of VHH D03 was reduced by substitutions at amino acids T416, I422, G523 and T526 of the HCV polyprotein, while binding of VHH B11 was reduced by mutations at I422, N540 and R614. The substitutions that resulted in greater than 50% reduction in binding of D03 were N415A, G523A and T526A for D03 and only N540A of the HCV polyprotein for B11. This mapped the epitope of VHH D03 to residues overlapping the previously defined CD81 binding region consistent with the inhibition of CD81 binding by VHH D03.

### REFERENCES

Allander, T., et al. (2000). J Gen Virol 81, 2451-2459.
Backovic, M., et al. (2010). Protein Eng Des Sel 23, 169-74.
Bankwitz, D., et al. (2010). Journal of Virology 84, 5751-5763.
Blight, K.J., et al. (2002). J Virol 76, 13001-13014.
Bricogne, G., et al. (2010). BUSTER version 2.9., Cambridge, United Kingdom, Global Phasing Ltd.
Brimacombe, C.L., et al. (2011). Journal of Virology 85, 596-605.
Cardoso, D.F., et al. (2000). Scand J Immunol 51, 337-344.
Collaborative Computational Project (1994). The CCP4 suite: programs for protein crystallography. Acta Crystallogr D Biol Crystallogr 50, 760-763.
Coppieters, K.T., et al. (2006). Arthritis Rheum 54, 1856-66.
Dowd, K.A., et al. (2009). Gastroenterology 136, 2377-2386.
Emsley, P., et al. (2010). Acta Crystallogr D Biol Crystallogr 66, 486-501.
Evans, P. (2005). Acta Crystallogr D Biol Crystallogr 62, 72-82.
Farid, S.S., (2007). J Chromatogr B Analyt Technol Biomed Life Sci 848, 8-18.
Féray, C., et al. (1998). Ann Intern Med 128, 810-816.
Forns, X., et al. (2000). Proc Natl Acad Sci U S A 97, 13318-13323.
Forsman, A., et al. (2008). Journal of Virology 82, 12069-12081.
Gilmartin, A.A., et al. (2012). Protein Eng Des Sel 25, 59-66.
Hadlock, K.G., et al. (2000). J Virol 74, 10407-16.
Hamers-Casterman, C., et al. (1993). Nature 363, 446-448.
Harmsen, M., et al. (2005). Vaccine 23, 4926-34.
Harmsen, M., et al. (2007). Veterinary Microbiology 120, 193-206.
Harmsen, M.M., and De Haard, H.J. (2007). Appl Microbiol Biotechnol 77, 13-22.
Hultberg, A., et al. (2011). PLoS ONE 6, e17665.
Johansson, D.X., et al. (2007). Proc Natl Acad Sci USA 104, 16269-16274.
Inchauspe, G., et al. (1991). Proc Natl Acad Sci U S A. 88, 10292-10296.
Kabsch, W., (1988). J Appl Crystallogr, 67-72.
Kato, T., et al. (2001) J Med Virol 64, 334-339.
Kitadokoro, K., et al. (2001). Acta Crystallogr D Biol Crystallogr 57, 156-8.
Kolykhalov, A.A., et al., (1997). Science 277, 570-574.
Krey, T., et al. (2010). PLoS Pathog 6, e1000762.
Kwo, P.Y., et al. (2010). The Lancet 376, 705-716.
Lafaye, P., et al. (1995). Res Immunol 146, 373-382.
Lafaye, P., et al. (2009). Mol Immunol 46, 695-704.
Lavillette, D., et al. (2005). Hepatology 41, 265-274.
Law, M., et al. (2007). Nat Med 14, 25-27.
Lefranc, M.-P., et al. (2009). Nucleic Acids Res 37, D1006-1012.
Lindenbach, B.D., et al. (2005). Science 309, 623-626.
McCaffrey, K., et al. (2007). J Virol 81, 9584-9590.
McCoy, A.J., et al. (2007). J Appl Crystallogr 40, 658-674.
McCoy, L.E., et al. (2012). Journal of Experimental Medicine 1-13.
McHutchison, J.G., et al. (2010). New England Journal of Medicine 362, 1292-1303.
Meunier, J.-C., et al. (2011). J Infect Dis 204,1186-90.
Muyldermans, S., (2001). J Biotechnol 74, 277-302.
Ndongo, N., et al. (2010). Hepatology 52, 1531-1542.
Nguyen, V.K., et al. (2000). The EMBO Journal 19, 921-930.
Owsianka, A., et al. (2001). J Gen Virol 82, 1877-83.
Owsianka, A., et al. 2005. Journal of Virology 79, 11095-104.
Owsianka, A.M., et al. (2006). J Virol 80, 8695-8704.
Pestka, J.M., et al. (2007). Proc Natl Acad Sci USA 104, 6025-6030.
Prentoe, J., et al. (2011). Journal of Virology 85, 2224-2234.
Reed, L.J., and Muench, H. (1938). The American Journal of Hygiene 24, 493-497.
Stamataki, Z.S., et al (2007). Vaccine 25, 7773-84.
Sarrazin, C., et al. (2012). Journal ofHepatology 56 Suppl 1, S88-100.
Simmonds, P., et al. (2005). Hepatology (Baltimore, Md) 42, 962-73.
Tarr, A.W., et al. (2007). Methods Mol Biol 379, 177-197.
Tarr, A.W., et al. (2011). J Virol 85, 4246-4257.
Tarr, A.W., et al. (2012). Journal of Virology 86, 2739-2749.
Thys, B., et al. (2010). Antiviral Research 87, 257-264.
van der Vaart, J.M., et al. (2006). Vaccine 24, 4130-4137.
Vanlandschoot, P., et al. (2011). Antiviral Research 92, 389-407.
Timpe, J.M., et al. (2008). Hepatology 47, 17-24.
Verna, E.C., and Brown, R.S., Jr. (2008). Clin Liver Dis 12, 637-659, ix-x.
Vincke, C., et al. (2009). J Biol Chem 284, 3273-84.
Vu, K.B., et al. (1997). Mol Immunol 34, 1121-1131.
Welsch, C., et al. (2012). Gut 61 Suppl 1, i36-i46.
Wernery, U., (2001). J Vet Med B Infect Dis Vet Public Health 48, 561-568.
Youn, J.-W., et al. (2005). Hepatology 42, 1429-1436.

## Claims

1. An isolated neutralizing antibody or fragment thereof directed against the ectodomain of a Hepatitis C virus glycoprotein E2, **characterized in that** said antibody and fragment thereof bind the contacting amino acid residues G523 and T526 of said Hepatitis C virus glycoprotein E2, in reference to the amino acid sequence of the polyprotein of the Hepatitis C virus strain H77 genotype 1a of SEQ ID NO: 7.

2. The isolated neutralizing antibody or fragment thereof according to claim 1, **characterized in that** they does not bind amino acid residues P525, W529, G530, D535 and N540 of said Hepatitis C virus glycoprotein E2, in reference to the amino acid sequence of the polyprotein of the Hepatitis C virus strain H77 genotype 1a of SEQ ID NO: 7.

3. The isolated neutralizing antibody or fragment thereof according to claim 1 or claim 2, **characterized in that** they comprise at least one Complementarity Determining Region (CDR) from VHH D03 of SEQ ID NO: 2, selected from the group consisting of SEQ ID NO: 31 (CDR1 from VHH D03), SEQ ID NO: 32 (CDR2 from VHH D03) and SEQ ID NO: 33 (CDR3 from VHH D03).

4. The isolated antibody fragment according to any one of claims 1 to 3, **characterized in that** it is a variable antigen-binding domain from a camelid heavy-chain antibody (VHH).

5. The isolated antibody fragment according to claim 3 or claim 4, **characterized in that** it comprises or consists of the amino acid sequence SEQ ID NO: 1.

6. The isolated antibody fragment according to claim 4 or claim 5, **characterized in that** it consists of the amino acid sequence SEQ ID NO: 2 (VHH D03).

7. The isolated antibody fragment according to any one of claims 4 to 6, **characterised in that** said VHH is obtainable by the method comprising the steps of:
(a) immunizing a camelid with a Hepatitis C virus glycoprotein E2 (HCV E2) or the ectodomain of a Hepatitis C virus glycoprotein E2 or with the ectodomain of a Hepatitis C virus glycoprotein E2 lacking the HVR1 domain,
(b) isolating peripheral lymphocytes of the immunized camelid, obtaining the total RNA and synthesizing the corresponding cDNAs,
(c) constructing a library of cDNA fragments encoding VHH,
(d) transcribing the VHH-encoding cDNAs obtained in step (c) to mRNA, and
(e) expressing the VHH in an expression vector.

8. The isolated neutralizing antibody or fragment thereof according to any one of claims 1 to 3, **characterized in that** it is in a form of a chimeric antibody, comprising:
- at least one CDR from VHH D03 (SEQ ID NO: 2), selected from the group consisting of SEQ ID NO: 31 (CDR1), SEQ ID NO: 32 (CDR2), SEQ ID NO: 33 (CDR3), and
- at least one CDR from VHH C09 (SEQ ID NO: 3), selected from the group consisting of SEQ ID NO: 34 (CDR1), SEQ ID NO: 35 (CDR2) and SEQ ID NO: 36 (CDR3).

9. An isolated polypeptide, **characterized in that** it comprises an antibody fragment as defined in any one of claims 1 to 8.

10. An isolated polynucleotide, **characterized in that** it encodes an antibody fragment or a polypeptide as defined in any one of claims 1 to 9.

11. A recombinant expression cassette, **characterized in that** it comprises a polynucleotide according to claim 10, under control of a transcriptional promoter.

12. A recombinant vector, **characterized in that** it comprises a polynucleotide according to claim 10 or a recombinant expression cassette according to claim 11.

13. A host cell, **characterized in that** it contains a recombinant expression cassette of claim 11 or a recombinant vector of claim 12.

14. A pharmaceutical composition, **characterized in that** it comprises a neutralizing antibody or fragment thereof as defined in any one of claims 1 to 8 or a polypeptide as defined in claim 9 or a recombinant vector according to claim 12, and a pharmaceutically acceptable carrier.

15. A diagnostic agent, **characterized in that** it comprises a neutralizing antibody or fragment thereof as defined in any one of claims 1 to 8 or a polypeptide as defined in claim 9, linked, directly or indirectly, covalently or non-covalently to a detectable marker.

16. The diagnostic agent according to claim 15, **characterized in that** the detectable marker is selected from the group consisting of enzymes, fluorophores, heavy metal chelates and radioisotopes.

17. A kit for diagnosing or monitoring, in a subject, a Hepatitis C virus infection, **characterized in that** it comprises a neutralizing antibody or fragment thereof as defined in any one of claims 1 to 8 or a polypeptide as defined in claim 9 or a diagnostic agent as defined in claims 15 or 16, and an appropriate diagnostic reagent.

18. An *in vitro* method for diagnosing a Hepatitis C virus infection in a subject, **characterized in that** it comprises the steps of:
a) contacting *in vitro* an appropriate biological sample from said subject with a neutralizing antibody or fragment thereof as defined in any one of claims 1 to 8 or a polypeptide as defined in claim 9 or a diagnostic agent as defined in claims 15 or 16, and
b) determining the presence or the absence of a HCV envelope glycoprotein E2 in said biological sample, the presence of said HCV envelope glycoprotein E2 indicating that said subject has Hepatitis C virus infection.

19. An *in vitro* method for monitoring the progression or regression of a Hepatitis C virus infection in a subject, **characterized in that** it comprises the steps of:
a) contacting *in vitro* an appropriate biological sample from said subject with a neutralizing antibody or fragment thereof as defined in any one of claims 1 to 8 or a polypeptide as defined in claim 9 or a diagnostic agent as defined in claims 15 or 16,
b) determining the amount of HCV envelope glycoprotein E2 in said biological sample, and
c) comparing the amount determined in step (b) with the amount of HCV envelope glycoprotein E2 previously obtained for said subject,
a significant increase in amount of HCV envelope glycoprotein E2 constituting a marker of the progression of said HCV infection and a significant decrease of HCV envelope glycoprotein E2 constituting a marker of the regression of said HCV infection.

20. An antigen consisting of a Hepatitis C virus E2 ectodomain lacking the hypervariable region 1 (HVR1), corresponding to amino acids 412-715 of the polyprotein of said HCV in reference to HCV strain H77c of SEQ ID NO: 7.


SEQUENCE LISTING

<110>  INSTITUT PASTEUR
       KREY, Thomas
       TARR, Alexander W.
       LAFAYE, Pierre
       BALL, Jonathan K.
       REY, Felix A.
       JESTIN, Jean-Luc

<120>  NEW NEUTRALIZING ANTIBODIES DIRECTED AGAINST HEPATITIS C VIRUS

<130>  XRN/cc-F226/195

<160>  36

<170>  PatentIn version 3.5

<210>  1
<211>  130
<212>  PRT
<213>  Artificial Sequence

<220>
<223>  Consensus sequence


<220>
<221>  VARIANT
<222>  (7)..(7)
<223>  replace by nothing

<220>
<221>  VARIANT
<222>  (12)..(12)
<223>  replace by Ala

<220>
<221>  VARIANT
<222>  (23)..(23)
<223>  replace by Glu

<220>
<221>  VARIANT
<222>  (28)..(28)
<223>  replace by Ser

<220>
<221>  VARIANT
<222>  (29)..(29)
<223>  replace by nothing

<220>
<221>  VARIANT
<222>  (30)..(30)
<223>  replace by Val

<220>
<221>  VARIANT
<222>  (31)..(31)
<223>  replace by Asn

<220>
<221>  VARIANT
<222>  (32)..(32)

<223>  replace by Leu

<220>
<221>  VARIANT
<222>  (33)..(33)
<223>  replace by Asp

<220>
<221>  VARIANT
<222>  (34)..(34)
<223>  replace by Thr

<220>
<221>  VARIANT
<222>  (52)..(52)
<223>  replace by Val

<220>
<221>  VARIANT
<222>  (53)..(53)
<223>  replace by Ser

<220>
<221>  VARIANT
<222>  (54)..(54)
<223>  replace by Glu

<220>
<221>  VARIANT
<222>  (55)..(55)
<223>  replace by Ser

<220>
<221>  VARIANT
<222>  (58)..(58)
<223>  replace by Lys

<220>
<221>  VARIANT
<222>  (59)..(59)
<223>  replace by Glu

<220>
<221>  VARIANT
<222>  (62)..(62)
<223>  replace by Thr

<220>
<221>  VARIANT
<222>  (67)..(67)
<223>  replace by Gly

<220>
<221>  VARIANT
<222>  (71)..(71)
<223>  replace by Ile

<220>
<221>  VARIANT
<222>  (80)..(80)
<223>  replace by Met

<220>
<221>  VARIANT
<222>  (85)..(85)

<223>  replace by Asp

<220>
<221>  VARIANT
<222>  (88)..(88)
<223>  replace by Asn

<220>
<221>  VARIANT
<222>  (89)..(89)
<223>  replace by Ala

<220>
<221>  VARIANT
<222>  (98)..(98)
<223>  replace by Val

<220>
<221>  VARIANT
<222>  (101)..(101)
<223>  replace by Ser

<220>
<221>  VARIANT
<222>  (102)..(102)
<223>  replace by nothing

<220>
<221>  VARIANT
<222>  (103)..(103)
<223>  replace by Ala

<220>
<221>  VARIANT
<222>  (104)..(104)
<223>  replace by Gln

<220>
<221>  VARIANT
<222>  (106)..(106)
<223>  replace by Gln

<220>
<221>  VARIANT
<222>  (107)..(107)
<223>  replace by Pro

<220>
<221>  VARIANT
<222>  (108)..(108)
<223>  replace by Pro

<220>
<221>  VARIANT
<222>  (109)..(109)
<223>  replace by Tyr

<220>
<221>  VARIANT
<222>  (110)..(110)
<223>  replace by Ser

<220>
<221>  VARIANT
<222>  (112)..(112)

<223>  replace by nothing

<220>
<221>  VARIANT
<222>  (113)..(113)
<223>  replace by Asn

<220>
<221>  VARIANT
<222>  (114)..(114)
<223>  replace by Glu

<220>
<221>  VARIANT
<222>  (115)..(115)
<223>  replace by Asp

<220>
<221>  VARIANT
<222>  (116)..(116)
<223>  replace by Glu

<220>
<221>  VARIANT
<222>  (117)..(117)
<223>  replace by Tyr

<220>
<221>  VARIANT
<222>  (118)..(118)
<223>  replace by Asp

<220>
<221>  VARIANT
<222>  (119)..(119)
<223>  replace by Tyr

<400>  1

Glu Val Gln Leu Gln Ala Ser Gly Gly Gly Leu Val Gln Pro Gly Gly
1               5                   10                  15

Ser Leu Arg Leu Ser Cys Thr Ala Ser Gly Phe Thr Leu Asp Asp Tyr
            20                  25                  30

Tyr Ala Ile Gly Trp Phe Arg Gln Ala Pro Gly Lys Glu Arg Glu Gly
        35                  40                  45

Val Ser Cys Ile Thr Asn Phe Asp Gly Gly Thr Tyr Tyr Ala Asp Ser
    50                  55                  60

Val Lys Ser Arg Phe Thr Met Ser Arg Asp Asn Ala Lys Asn Thr Val
65                  70                  75                  80

Tyr Leu Gln Met Asn Ser Leu Lys Pro Glu Asp Thr Ala Val Tyr Tyr
                85                  90                  95

Cys Ala Ala Asp Lys Phe Gly Leu Cys Ser Trp Leu Arg Ala Gly Trp
            100                 105                 110

Gly Lys Val Thr Phe Gly Ser Trp Gly Gln Gly Thr Gln Val Thr Val
        115                 120                 125

Ser Ser
    130

<210>  2
<211>  127
<212>  PRT
<213>  Lama pacos

<400>  2

Glu Val Gln Leu Gln Ala Ser Gly Gly Gly Leu Val Gln Pro Gly Gly
1               5                   10                  15

Ser Leu Arg Leu Ser Cys Thr Ala Ser Gly Phe Thr Asp Asp Tyr Tyr
            20                  25                  30

Ala Ile Gly Trp Phe Arg Gln Ala Pro Gly Lys Glu Arg Glu Gly Val
        35                  40                  45

Ser Cys Ile Thr Asn Phe Asp Gly Gly Thr Tyr Tyr Ala Asp Ser Val
    50                  55                  60

Lys Ser Arg Phe Thr Met Ser Arg Asp Asn Ala Lys Asn Thr Val Tyr
65                  70                  75                  80

Leu Gln Met Asn Ser Leu Lys Pro Glu Asp Thr Ala Val Tyr Tyr Cys
                85                  90                  95

Ala Ala Asp Lys Gly Leu Cys Ser Trp Leu Arg Ala Gly Gly Lys Val
            100                 105                 110

Thr Phe Gly Ser Trp Gly Gln Gly Thr Gln Val Thr Val Ser Ser
        115                 120                 125

<210>  3
<211>  129
<212>  PRT
<213>  Lama pacos

<400>  3

Glu Val Gln Leu Gln Ala Gly Gly Gly Leu Ala Gln Pro Gly Gly Ser
1               5                   10                  15

Leu Arg Leu Ser Cys Glu Ala Ser Gly Phe Ser Leu Val Asn Leu Asp
            20                  25                  30

Thr Ile Gly Trp Phe Arg Gln Ala Pro Gly Lys Glu Arg Glu Gly Val

35                  40                  45

Ser Cys Val Ser Glu Ser Asp Gly Lys Glu Tyr Tyr Thr Asp Ser Val
    50                  55                  60

Lys Gly Arg Phe Thr Ile Ser Arg Asp Asn Ala Lys Asn Thr Met Tyr
65                  70                  75                  80

Leu Gln Met Asp Ser Leu Asn Ala Glu Asp Thr Ala Val Tyr Tyr Cys
                85                  90                  95

Val Ala Asp Ser Phe Ala Gln Cys Gln Pro Pro Tyr Ser Gly Trp Asn
            100                 105                 110

Glu Asp Glu Tyr Asp Tyr Trp Gly Gln Gly Thr Gln Val Thr Val Ser
        115                 120                 125

Ser

<210>  4
<211>  126
<212>  PRT
<213>  Lama pacos

<400>  4

Glu Glu Gln Leu Gln Ala Ser Gly Gly Gly Leu Val Gln Ala Gly Gly
1               5                   10                  15

Ser Leu Arg Leu Ser Cys Thr Ala Ser Glu Arg Ala Leu Ser Gly Leu
            20                  25                  30

Ser Val Gly Trp Phe Arg Gln Ala Pro Gly Lys Glu Arg Glu Leu Val
        35                  40                  45

Ala Ala Leu His Arg Asp Gly Asp Met Met Tyr Tyr Ala Asp Phe Glu
    50                  55                  60

Met Gly Arg Phe Thr Ile Ser Arg Asp Asn Ala Lys Asn Thr Val Asn
65                  70                  75                  80

Leu Leu Met Asn Ser Leu Lys Pro Glu Asp Thr Ala Val Tyr Tyr Cys
                85                  90                  95

Ala Ala Ser Thr Gln Lys Tyr Tyr Glu Asp Leu Arg Leu Leu Arg Asp
            100                 105                 110

Tyr Asp Tyr Trp Gly Gln Gly Thr Gln Val Thr Val Ser Ser
        115                 120                 125

<210>  5
<211>  123
<212>  PRT
<213>  Lama pacos

<400>  5

Glu Val Gln Leu Gln Ala Ser Gly Gly Gly Leu Val Gln Val Gly Gly
1               5                   10                  15

Ser Leu Arg Leu Ser Cys Val Ala Arg Ser Pro Ser Thr Leu Ala Ile
            20                  25                  30

Gly Trp Phe Arg Gln Ala Pro Gly Lys Glu Arg Glu Phe Val Ala Ala
        35                  40                  45

Ile Ser Trp Ser Gly Gly Thr Thr His Tyr Ala Asn Ser Ala Lys Gly
    50                  55                  60

Arg Phe Thr Ile Ser Arg Asp Asn Ala Lys Asn Thr Ile Tyr Leu Gln
65                  70                  75                  80

Met Asn Met Leu Lys Pro Glu Asp Thr Ala Val Tyr Tyr Cys Ala Ala
                85                  90                  95

Asp Arg Phe Gly Ser Gly Ala Ala Leu Thr Arg Arg Val Ala Asp Asp
            100                 105                 110

Trp Gly Gln Gly Ile Gln Val Thr Val Ser Ser
        115                 120


<210>  6
<211>  41
<212>  DNA
<213>  Artificial Sequence

<220>
<223>  Primer

<400>  6
ggactagttg cggccgctgg ttgtggtttt ggtgtcttgg g                          41


<210>  7
<211>  3011
<212>  PRT
<213>  Hepatitis C virus

<400>  7

Met Ser Thr Asn Pro Lys Pro Gln Arg Lys Thr Lys Arg Asn Thr Asn
1               5                   10                  15

Arg Arg Pro Gln Asp Val Lys Phe Pro Gly Gly Gly Gln Ile Val Gly
            20                  25                  30

Gly Val Tyr Leu Leu Pro Arg Arg Gly Pro Arg Leu Gly Val Arg Ala
        35                  40                  45

Thr Arg Lys Thr Ser Glu Arg Ser Gln Pro Arg Gly Arg Arg Gln Pro
    50                  55                  60

Ile Pro Lys Ala Arg Arg Pro Glu Gly Arg Thr Trp Ala Gln Pro Gly
65                  70                  75                  80

Tyr Pro Trp Pro Leu Tyr Gly Asn Glu Gly Cys Gly Trp Ala Gly Trp
                85                  90                  95

Leu Leu Ser Pro Arg Gly Ser Arg Pro Ser Trp Gly Pro Thr Asp Pro
            100                 105                 110

Arg Arg Arg Ser Arg Asn Leu Gly Lys Val Ile Asp Thr Leu Thr Cys
        115                 120                 125

Gly Phe Ala Asp Leu Met Gly Tyr Ile Pro Leu Val Gly Ala Pro Leu
    130                 135                 140

Gly Gly Ala Ala Arg Ala Leu Ala His Gly Val Arg Val Leu Glu Asp
145                 150                 155                 160

Gly Val Asn Tyr Ala Thr Gly Asn Leu Pro Gly Cys Ser Phe Ser Ile
                165                 170                 175

Phe Leu Leu Ala Leu Leu Ser Cys Leu Thr Val Pro Ala Ser Ala Tyr
            180                 185                 190

Gln Val Arg Asn Ser Ser Gly Leu Tyr His Val Thr Asn Asp Cys Pro
        195                 200                 205

Asn Ser Ser Val Val Tyr Glu Ala Ala Asp Ala Ile Leu His Thr Pro
    210                 215                 220

Gly Cys Val Pro Cys Val Arg Glu Gly Asn Ala Ser Arg Cys Trp Val
225                 230                 235                 240

Ala Val Thr Pro Thr Val Ala Thr Arg Asp Gly Lys Leu Pro Thr Thr
                245                 250                 255

Gln Leu Arg Arg His Ile Asp Leu Leu Val Gly Ser Ala Thr Leu Cys
            260                 265                 270

Ser Ala Leu Tyr Val Gly Asp Leu Cys Gly Ser Val Phe Leu Val Gly
        275                 280                 285

Gln Leu Phe Thr Phe Ser Pro Arg His His Trp Thr Thr Gln Asp Cys
    290                 295                 300

Asn Cys Ser Ile Tyr Pro Gly His Ile Thr Gly His Arg Met Ala Trp
305                 310                 315                 320

Asn Met Met Met Asn Trp Ser Pro Thr Ala Ala Leu Val Val Ala Gln
                325                 330                 335

Leu Leu Arg Ile Pro Gln Ala Ile Met Asp Met Ile Ala Gly Ala His
            340                 345                 350

Trp Gly Val Leu Ala Gly Ile Lys Tyr Phe Ser Met Val Gly Asn Trp
        355                 360                 365

Ala Lys Val Leu Val Val Leu Leu Leu Phe Ala Gly Val Asp Ala Glu
    370                 375                 380

Thr His Val Thr Gly Gly Asn Ala Gly Arg Thr Thr Ala Gly Leu Val
385                 390                 395                 400

Gly Leu Leu Thr Pro Gly Ala Lys Gln Asn Ile Gln Leu Ile Asn Thr
                405                 410                 415

Asn Gly Ser Trp His Ile Asn Ser Thr Ala Leu Asn Cys Asn Glu Ser
            420                 425                 430

Leu Asn Thr Gly Trp Leu Ala Gly Leu Phe Tyr Gln His Lys Phe Asn
        435                 440                 445

Ser Ser Gly Cys Pro Glu Arg Leu Ala Ser Cys Arg Arg Leu Thr Asp
    450                 455                 460

Phe Ala Gln Gly Trp Gly Pro Ile Ser Tyr Ala Asn Gly Ser Gly Leu
465                 470                 475                 480

Asp Glu Arg Pro Tyr Cys Trp His Tyr Pro Pro Arg Pro Cys Gly Ile
                485                 490                 495

Val Pro Ala Lys Ser Val Cys Gly Pro Val Tyr Cys Phe Thr Pro Ser
            500                 505                 510

Pro Val Val Val Gly Thr Thr Asp Arg Ser Gly Ala Pro Thr Tyr Ser
        515                 520                 525

Trp Gly Ala Asn Asp Thr Asp Val Phe Val Leu Asn Asn Thr Arg Pro
    530                 535                 540

Pro Leu Gly Asn Trp Phe Gly Cys Thr Trp Met Asn Ser Thr Gly Phe
545                 550                 555                 560

Thr Lys Val Cys Gly Ala Pro Pro Cys Val Ile Gly Gly Val Gly Asn
                565                 570                 575

Asn Thr Leu Leu Cys Pro Thr Asp Cys Phe Arg Lys Tyr Pro Glu Ala
            580                 585                 590

Thr Tyr Ser Arg Cys Gly Ser Gly Pro Arg Ile Thr Pro Arg Cys Met
        595                 600                 605

Val Asp Tyr Pro Tyr Arg Leu Trp His Tyr Pro Cys Thr Ile Asn Tyr
    610                 615                 620

Thr Ile Phe Lys Val Arg Met Tyr Val Gly Gly Val Glu His Arg Leu
625                 630                 635                 640

Glu Ala Ala Cys Asn Trp Thr Arg Gly Glu Arg Cys Asp Leu Glu Asp
                645                 650                 655

Arg Asp Arg Ser Glu Leu Ser Pro Leu Leu Leu Ser Thr Thr Gln Trp
            660                 665                 670

Gln Val Leu Pro Cys Ser Phe Thr Thr Leu Pro Ala Leu Ser Thr Gly
        675                 680                 685

Leu Ile His Leu His Gln Asn Ile Val Asp Val Gln Tyr Leu Tyr Gly
    690                 695                 700

Val Gly Ser Ser Ile Ala Ser Trp Ala Ile Lys Trp Glu Tyr Val Val
705                 710                 715                 720

Leu Leu Phe Leu Leu Leu Ala Asp Ala Arg Val Cys Ser Cys Leu Trp
                725                 730                 735

Met Met Leu Leu Ile Ser Gln Ala Glu Ala Ala Leu Glu Asn Leu Val
            740                 745                 750

Ile Leu Asn Ala Ala Ser Leu Ala Gly Thr His Gly Leu Val Ser Phe
        755                 760                 765

Leu Val Phe Phe Cys Phe Ala Trp Tyr Leu Lys Gly Arg Trp Val Pro
    770                 775                 780

Gly Ala Val Tyr Ala Leu Tyr Gly Met Trp Pro Leu Leu Leu Leu Leu
785                 790                 795                 800

Leu Ala Leu Pro Gln Arg Ala Tyr Ala Leu Asp Thr Glu Val Ala Ala

805                 810                 815

Ser Cys Gly Gly Val Val Leu Val Gly Leu Met Ala Leu Thr Leu Ser
        820                 825                 830

Pro Tyr Tyr Lys Arg Tyr Ile Ser Trp Cys Met Trp Trp Leu Gln Tyr
    835                 840                 845

Phe Leu Thr Arg Val Glu Ala Gln Leu His Val Trp Val Pro Pro Leu
    850                 855                 860

Asn Val Arg Gly Gly Arg Asp Ala Val Ile Leu Leu Thr Cys Val Val
865                 870                 875                 880

His Pro Ala Leu Val Phe Asp Ile Thr Lys Leu Leu Leu Ala Ile Phe
                885                 890                 895

Gly Pro Leu Trp Ile Leu Gln Ala Ser Leu Leu Lys Val Pro Tyr Phe
        900                 905                 910

Val Arg Val Gln Gly Leu Leu Arg Ile Cys Ala Leu Ala Arg Lys Ile
        915                 920                 925

Ala Gly Gly His Tyr Val Gln Met Ala Ile Ile Lys Leu Gly Ala Leu
    930                 935                 940

Thr Gly Thr Cys Val Tyr Asn His Leu Ala Pro Leu Arg Asp Trp Ala
945                 950                 955                 960

His Asn Gly Leu Arg Asp Leu Ala Val Ala Val Glu Pro Val Val Phe
                965                 970                 975

Ser Arg Met Glu Thr Lys Leu Ile Thr Trp Gly Ala Asp Thr Ala Ala
        980                 985                 990

Cys Gly Asp Ile Ile Asn Gly Leu  Pro Val Ser Ala Arg  Arg Gly Gln
        995                 1000                1005

Glu Ile  Leu Leu Gly Pro Ala  Asp Gly Met Val Ser  Lys Gly Trp
    1010                1015                1020

Arg Leu  Leu Ala Pro Ile Thr  Ala Tyr Ala Gln Gln  Thr Arg Gly
    1025                1030                1035

Leu Leu  Gly Cys Ile Ile Thr  Ser Leu Thr Gly Arg  Asp Lys Asn
    1040                1045                1050

Gln Val  Glu Gly Glu Val Gln  Ile Val Ser Thr Ala  Thr Gln Thr
    1055                1060                1065

Phe Leu Ala Thr Cys Ile Asn Gly Val Cys Trp Thr Val Tyr His
    1070                1075                1080

Gly Ala Gly Thr Arg Thr Ile Ala Ser Pro Lys Gly Pro Val Ile
    1085                1090                1095

Gln Thr Tyr Thr Asn Val Asp Gln Asp Leu Val Gly Trp Pro Ala
    1100                1105                1110

Pro Gln Gly Ser Arg Ser Leu Thr Pro Cys Thr Cys Gly Ser Ser
    1115                1120                1125

Asp Leu Tyr Leu Val Thr Arg His Ala Asp Val Ile Pro Val Arg
    1130                1135                1140

Arg Arg Gly Asp Ser Arg Gly Ser Leu Leu Ser Pro Arg Pro Ile
    1145                1150                1155

Ser Tyr Leu Lys Gly Ser Ser Gly Gly Pro Leu Leu Cys Pro Thr
    1160                1165                1170

Gly His Ala Val Gly Leu Phe Arg Ala Ala Val Cys Thr Arg Gly
    1175                1180                1185

Val Ala Lys Ala Val Asp Phe Ile Pro Val Glu Asn Leu Glu Thr
    1190                1195                1200

Thr Met Arg Ser Pro Val Phe Thr Asp Asn Ser Ser Pro Pro Ala
    1205                1210                1215

Val Pro Gln Ser Phe Gln Val Ala His Leu His Ala Pro Thr Gly
    1220                1225                1230

Ser Gly Lys Ser Thr Lys Val Pro Ala Ala Tyr Ala Ala Lys Gly
    1235                1240                1245

Tyr Lys Val Leu Val Leu Asn Pro Ser Val Ala Ala Thr Leu Gly
    1250                1255                1260

Phe Gly Ala Tyr Met Ser Lys Ala His Gly Val Asp Pro Asn Ile
    1265                1270                1275

Arg Thr Gly Val Arg Thr Ile Thr Thr Gly Ser Pro Ile Thr Tyr
    1280                1285                1290

Ser Thr Tyr Gly Lys Phe Leu Ala Asp Ala Gly Cys Ser Gly Gly
    1295                1300                1305

Ala Tyr Asp Ile Ile Ile Cys Asp Glu Cys His Ser Thr Asp Ala
    1310                1315                1320

Thr Ser Ile Ser Gly Ile Gly Thr Val Leu Asp Gln Ala Glu Thr
    1325                1330                1335

Ala Gly Ala Arg Leu Val Val Leu Ala Thr Ala Thr Pro Pro Gly
    1340                1345                1350

Ser Val Thr Val Ser His Pro Asn Ile Glu Glu Val Ala Leu Ser
    1355                1360                1365

Thr Thr Gly Glu Ile Pro Phe Tyr Gly Lys Ala Ile Pro Leu Glu
    1370                1375                1380

Val Ile Lys Gly Gly Arg His Leu Ile Phe Cys His Ser Lys Lys
    1385                1390                1395

Lys Cys Asp Glu Leu Ala Ala Lys Leu Val Ala Leu Gly Ile Asn
    1400                1405                1410

Ala Val Ala Tyr Tyr Arg Gly Leu Asp Val Ser Val Ile Pro Thr
    1415                1420                1425

Ser Gly Asp Val Val Val Val Ser Thr Asp Ala Leu Met Thr Gly
    1430                1435                1440

Phe Thr Gly Asp Phe Asp Ser Val Ile Asp Cys Asn Thr Cys Val
    1445                1450                1455

Thr Gln Thr Val Asp Phe Ser Leu Asp Pro Thr Phe Thr Ile Glu
    1460                1465                1470

Thr Thr Thr Leu Pro Gln Asp Ala Val Ser Arg Thr Gln Arg Arg
    1475                1480                1485

Gly Arg Thr Gly Arg Gly Lys Pro Gly Ile Tyr Arg Phe Val Ala
    1490                1495                1500

Pro Gly Glu Arg Pro Ser Gly Met Phe Asp Ser Ser Val Leu Cys
    1505                1510                1515

Glu Cys Tyr Asp Ala Gly Cys Ala Trp Tyr Glu Leu Thr Pro Ala
    1520                1525                1530

Glu Thr Thr Val Arg Leu Arg Ala Tyr Met Asn Thr Pro Gly Leu
    1535                1540                1545

Pro Val Cys Gln Asp His Leu Gly Phe Trp Glu Gly Val Phe Thr
    1550                1555                1560

Gly Leu Thr His Ile Asp Ala His Phe Leu Ser Gln Thr Lys Gln
    1565                1570                1575

Ser Gly Glu Asn Phe Pro Tyr Leu Val Ala Tyr Gln Ala Thr Val
    1580                1585                1590

Cys Ala Arg Ala Gln Ala Pro Pro Pro Ser Trp Asp Gln Met Arg
    1595                1600                1605

Lys Cys Leu Ile Arg Leu Lys Pro Thr Leu His Gly Pro Thr Pro
    1610                1615                1620

Leu Leu Tyr Arg Leu Gly Ala Val Gln Asn Glu Val Thr Leu Thr
    1625                1630                1635

His Pro Ile Thr Lys Tyr Ile Met Thr Cys Met Ser Ala Asp Leu
    1640                1645                1650

Glu Val Val Thr Ser Thr Trp Val Leu Val Gly Gly Val Leu Ala
    1655                1660                1665

Ala Leu Ala Ala Tyr Cys Leu Ser Thr Gly Cys Val Val Ile Val
    1670                1675                1680

Gly Arg Ile Val Leu Ser Gly Lys Pro Ala Ile Ile Pro Asp Arg
    1685                1690                1695

Glu Val Leu Tyr Gln Glu Phe Asp Glu Met Glu Glu Cys Ser Gln
    1700                1705                1710

His Leu Pro Tyr Ile Glu Gln Gly Met Met Leu Ala Glu Gln Phe
    1715                1720                1725

Lys Gln Lys Ala Leu Gly Leu Leu Gln Thr Ala Ser Arg His Ala
    1730                1735                1740

Glu Val Ile Thr Pro Ala Val Gln Thr Asn Trp Gln Lys Leu Glu
    1745                1750                1755

Val Phe Trp Ala Lys His Met Trp Asn Phe Ile Ser Gly Ile Gln
    1760                1765                1770

Tyr Leu Ala Gly Leu Ser Thr Leu Pro Gly Asn Pro Ala Ile Ala
    1775                1780                1785

Ser Leu Met Ala Phe Thr Ala Ala Val Thr Ser Pro Leu Thr Thr

1790                1795                1800

Gly Gln  Thr Leu Leu Phe Asn  Ile Leu Gly Gly Trp  Val Ala Ala
    1805                1810                1815

Gln Leu  Ala Ala Pro Gly Ala  Ala Thr Ala Phe Val  Gly Ala Gly
    1820                1825                1830

Leu Ala  Gly Ala Ala Leu Asp  Ser Val Gly Leu Gly  Lys Val Leu
    1835                1840                1845

Val Asp  Ile Leu Ala Gly Tyr  Gly Ala Gly Val Ala  Gly Ala Leu
    1850                1855                1860

Val Ala  Phe Lys Ile Met Ser  Gly Glu Val Pro Ser  Thr Glu Asp
    1865                1870                1875

Leu Val  Asn Leu Leu Pro Ala  Ile Leu Ser Pro Gly  Ala Leu Ala
    1880                1885                1890

Val Gly  Val Val Phe Ala Ser  Ile Leu Arg Arg Arg  Val Gly Pro
    1895                1900                1905

Gly Glu  Gly Ala Val Gln Trp  Met Asn Arg Leu Ile  Ala Phe Ala
    1910                1915                1920

Ser Arg  Gly Asn His Val Ser  Pro Thr His Tyr Val  Pro Glu Ser
    1925                1930                1935

Asp Ala  Ala Ala Arg Val Thr  Ala Ile Leu Ser Ser  Leu Thr Val
    1940                1945                1950

Thr Gln  Leu Leu Arg Arg Leu  His Gln Trp Ile Ser  Ser Glu Cys
    1955                1960                1965

Thr Thr  Pro Cys Ser Gly Ser  Trp Leu Arg Asp Ile  Trp Asp Trp
    1970                1975                1980

Ile Cys  Glu Val Leu Ser Asp  Phe Lys Thr Trp Leu  Lys Ala Lys
    1985                1990                1995

Leu Met  Pro Gln Leu Pro Gly  Ile Pro Phe Val Ser  Cys Gln Arg
    2000                2005                2010

Gly Tyr  Arg Gly Val Trp Arg  Gly Asp Gly Ile Met  His Thr Arg
    2015                2020                2025

Cys His  Cys Gly Ala Glu Ile  Thr Gly His Val Lys  Asn Gly Thr
    2030                2035                2040

Met Arg Ile Val Gly Pro Arg Thr Cys Lys Asn Met Trp Ser Gly
    2045                2050                2055

Thr Phe Phe Ile Asn Ala Tyr Thr Thr Gly Pro Cys Thr Pro Leu
    2060                2065                2070

Pro Ala Pro Asn Tyr Lys Phe Ala Leu Trp Arg Val Ser Ala Glu
    2075                2080                2085

Glu Tyr Val Glu Ile Arg Arg Val Gly Asp Phe His Tyr Val Ser
    2090                2095                2100

Gly Met Thr Thr Asp Asn Leu Lys Cys Pro Cys Gln Ile Pro Ser
    2105                2110                2115

Pro Glu Phe Phe Thr Glu Leu Asp Gly Val Arg Leu His Arg Phe
    2120                2125                2130

Ala Pro Pro Cys Lys Pro Leu Leu Arg Glu Glu Val Ser Phe Arg
    2135                2140                2145

Val Gly Leu His Glu Tyr Pro Val Gly Ser Gln Leu Pro Cys Glu
    2150                2155                2160

Pro Glu Pro Asp Val Ala Val Leu Thr Ser Met Leu Thr Asp Pro
    2165                2170                2175

Ser His Ile Thr Ala Glu Ala Ala Gly Arg Arg Leu Ala Arg Gly
    2180                2185                2190

Ser Pro Pro Ser Met Ala Ser Ser Ser Ala Ser Gln Leu Ser Ala
    2195                2200                2205

Pro Ser Leu Lys Ala Thr Cys Thr Ala Asn His Asp Ser Pro Asp
    2210                2215                2220

Ala Glu Leu Ile Glu Ala Asn Leu Leu Trp Arg Gln Glu Met Gly
    2225                2230                2235

Gly Asn Ile Thr Arg Val Glu Ser Glu Asn Lys Val Val Ile Leu
    2240                2245                2250

Asp Ser Phe Asp Pro Leu Val Ala Glu Glu Asp Glu Arg Glu Val
    2255                2260                2265

Ser Val Pro Ala Glu Ile Leu Arg Lys Ser Arg Arg Phe Ala Pro
    2270                2275                2280

Ala Leu Pro Val Trp Ala Arg Pro Asp Tyr Asn Pro Leu Leu Val
    2285                2290                2295

Glu Thr Trp Lys Lys Pro Asp Tyr Glu Pro Pro Val Val His Gly
    2300                2305                2310

Cys Pro Leu Pro Pro Pro Arg Ser Pro Pro Val Pro Pro Pro Arg
    2315                2320                2325

Lys Lys Arg Thr Val Val Leu Thr Glu Ser Thr Leu Pro Thr Ala
    2330                2335                2340

Leu Ala Glu Leu Ala Thr Lys Ser Phe Gly Ser Ser Ser Thr Ser
    2345                2350                2355

Gly Ile Thr Gly Asp Asn Thr Thr Thr Ser Ser Glu Pro Ala Pro
    2360                2365                2370

Ser Gly Cys Pro Pro Asp Ser Asp Val Glu Ser Tyr Ser Ser Met
    2375                2380                2385

Pro Pro Leu Glu Gly Glu Pro Gly Asp Pro Asp Leu Ser Asp Gly
    2390                2395                2400

Ser Trp Ser Thr Val Ser Ser Gly Ala Asp Thr Glu Asp Val Val
    2405                2410                2415

Cys Cys Ser Met Ser Tyr Ser Trp Thr Gly Ala Leu Val Thr Pro
    2420                2425                2430

Cys Ala Ala Glu Glu Gln Lys Leu Pro Ile Asn Ala Leu Ser Asn
    2435                2440                2445

Ser Leu Leu Arg His His Asn Leu Val Tyr Ser Thr Thr Ser Arg
    2450                2455                2460

Ser Ala Cys Gln Arg Lys Lys Lys Val Thr Phe Asp Arg Leu Gln
    2465                2470                2475

Val Leu Asp Ser His Tyr Gln Asp Val Leu Lys Glu Val Lys Ala
    2480                2485                2490

Ala Ala Ser Lys Val Lys Ala Asn Leu Leu Ser Val Glu Glu Ala
    2495                2500                2505

Cys Ser Leu Ala Pro Pro His Ser Ala Lys Ser Lys Phe Gly Tyr
    2510                2515                2520

Gly Ala Lys Asp Val Arg Cys His Ala Arg Lys Ala Val Ala His
    2525                2530                2535

Ile Asn Ser Val Trp Lys Asp Leu Leu Glu Asp Ser Val Thr Pro
    2540                2545                2550

Ile Asp Thr Thr Ile Met Ala Lys Asn Glu Val Phe Cys Val Gln
    2555                2560                2565

Pro Glu Lys Gly Gly Arg Lys Pro Ala Arg Leu Ile Val Phe Pro
    2570                2575                2580

Asp Leu Gly Val Arg Val Cys Glu Lys Met Ala Leu Tyr Asp Val
    2585                2590                2595

Val Ser Lys Leu Pro Leu Ala Val Met Gly Ser Ser Tyr Gly Phe
    2600                2605                2610

Gln Tyr Ser Pro Gly Gln Arg Val Glu Phe Leu Val Gln Ala Trp
    2615                2620                2625

Lys Ser Lys Lys Thr Pro Met Gly Leu Ser Tyr Asp Thr Arg Cys
    2630                2635                2640

Phe Asp Ser Thr Val Thr Glu Ser Asp Ile Arg Thr Glu Glu Ala
    2645                2650                2655

Ile Tyr Gln Cys Cys Asp Leu Asp Pro Gln Ala Arg Val Ala Ile
    2660                2665                2670

Lys Ser Leu Thr Glu Arg Leu Tyr Val Gly Gly Pro Leu Thr Asn
    2675                2680                2685

Ser Arg Gly Glu Asn Cys Gly Tyr Arg Arg Cys Arg Ala Ser Arg
    2690                2695                2700

Val Leu Thr Thr Ser Cys Gly Asn Thr Leu Thr Arg Tyr Ile Lys
    2705                2710                2715

Ala Arg Ala Ala Cys Arg Ala Ala Gly Leu Gln Asp Cys Thr Met
    2720                2725                2730

Leu Val Cys Gly Asp Asp Leu Val Val Ile Cys Glu Ser Ala Gly
    2735                2740                2745

Val Gln Glu Asp Ala Ala Ser Leu Arg Ala Phe Thr Glu Ala Met
    2750                2755                2760

Thr Arg Tyr Ser Ala Pro Pro Gly Asp Pro Pro Gln Pro Glu Tyr

2765                2770                2775

Asp Leu Glu Leu Ile Thr Ser Cys Ser Ser Asn Val Ser Val Ala
    2780                2785                2790

His Asp Gly Ala Gly Lys Arg Val Tyr Tyr Leu Thr Arg Asp Pro
    2795                2800                2805

Thr Thr Pro Leu Ala Arg Ala Ala Trp Glu Thr Ala Arg His Thr
    2810                2815                2820

Pro Val Asn Ser Trp Leu Gly Asn Ile Ile Met Phe Ala Pro Thr
    2825                2830                2835

Leu Trp Ala Arg Met Ile Leu Met Thr His Phe Phe Ser Val Leu
    2840                2845                2850

Ile Ala Arg Asp Gln Leu Glu Gln Ala Leu Asn Cys Glu Ile Tyr
    2855                2860                2865

Gly Ala Cys Tyr Ser Ile Glu Pro Leu Asp Leu Pro Pro Ile Ile
    2870                2875                2880

Gln Arg Leu His Gly Leu Ser Ala Phe Ser Leu His Ser Tyr Ser
    2885                2890                2895

Pro Gly Glu Ile Asn Arg Val Ala Ala Cys Leu Arg Lys Leu Gly
    2900                2905                2910

Val Pro Pro Leu Arg Ala Trp Arg His Arg Ala Trp Ser Val Arg
    2915                2920                2925

Ala Arg Leu Leu Ala Arg Gly Gly Lys Ala Ala Ile Cys Gly Lys
    2930                2935                2940

Tyr Leu Phe Asn Trp Ala Val Arg Thr Lys Leu Lys Leu Thr Pro
    2945                2950                2955

Ile Thr Ala Ala Gly Arg Leu Asp Leu Ser Gly Trp Phe Thr Ala
    2960                2965                2970

Gly Tyr Ser Gly Gly Asp Ile Tyr His Ser Val Ser His Ala Arg
    2975                2980                2985

Pro Arg Trp Phe Trp Phe Cys Leu Leu Leu Leu Ala Ala Gly Val
    2990                2995                3000

Gly Ile Tyr Leu Leu Pro Asn Arg
    3005                3010

<210>  8
<211>  332
<212>  PRT
<213>  Hepatitis C virus

<400>  8

Glu Thr His Val Thr Gly Gly Asn Ala Gly Arg Thr Thr Ala Gly Leu
1               5                   10                  15

Val Gly Leu Leu Thr Pro Gly Ala Lys Gln Asn Ile Gln Leu Ile Asn
            20                  25                  30

Thr Asn Gly Ser Trp His Ile Asn Ser Thr Ala Leu Asn Cys Asn Glu
        35                  40                  45

Ser Leu Asn Thr Gly Trp Leu Ala Gly Leu Phe Tyr Gln His Lys Phe
    50                  55                  60

Asn Ser Ser Gly Cys Pro Glu Arg Leu Ala Ser Cys Arg Arg Leu Thr
65                  70                  75                  80

Asp Phe Ala Gln Gly Trp Gly Pro Ile Ser Tyr Ala Asn Gly Ser Gly
                85                  90                  95

Leu Asp Glu Arg Pro Tyr Cys Trp His Tyr Pro Pro Arg Pro Cys Gly
            100                 105                 110

Ile Val Pro Ala Lys Ser Val Cys Gly Pro Val Tyr Cys Phe Thr Pro
        115                 120                 125

Ser Pro Val Val Val Gly Thr Thr Asp Arg Ser Gly Ala Pro Thr Tyr
    130                 135                 140

Ser Trp Gly Ala Asn Asp Thr Asp Val Phe Val Leu Asn Asn Thr Arg
145                 150                 155                 160

Pro Pro Leu Gly Asn Trp Phe Gly Cys Thr Trp Met Asn Ser Thr Gly
                165                 170                 175

Phe Thr Lys Val Cys Gly Ala Pro Pro Cys Val Ile Gly Gly Val Gly
            180                 185                 190

Asn Asn Thr Leu Leu Cys Pro Thr Asp Cys Phe Arg Lys Tyr Pro Glu
        195                 200                 205

Ala Thr Tyr Ser Arg Cys Gly Ser Gly Pro Arg Ile Thr Pro Arg Cys
    210                 215                 220

Met Val Asp Tyr Pro Tyr Arg Leu Trp His Tyr Pro Cys Thr Ile Asn
225                 230                 235                 240

Tyr Thr Ile Phe Lys Val Arg Met Tyr Val Gly Gly Val Glu His Arg
                245                 250                 255

Leu Glu Ala Ala Cys Asn Trp Thr Arg Gly Glu Arg Cys Asp Leu Glu
            260                 265                 270

Asp Arg Asp Arg Ser Glu Leu Ser Pro Leu Leu Leu Ser Thr Thr Gln
        275                 280                 285

Trp Gln Val Leu Pro Cys Ser Phe Thr Thr Leu Pro Ala Leu Ser Thr
    290                 295                 300

Gly Leu Ile His Leu His Gln Asn Ile Val Asp Val Gln Tyr Leu Tyr
305                 310                 315                 320

Gly Val Gly Ser Ser Ile Ala Ser Trp Ala Ile Lys
                325                 330

<210>  9
<211>  336
<212>  PRT
<213>  Hepatitis C virus

<400>  9

His Thr Arg Leu Ile Gly Ser Val Ala Gly Arg Asn Thr Asp Thr Leu
1               5                   10                  15

Arg Ser Leu Phe Ala Thr Gly Ala Gln Gln Lys Ile Ser Leu Ile Asn
            20                  25                  30

Thr Asn Gly Ser Trp His Ile Asn Arg Thr Ala Leu Asn Cys Asn Asp
        35                  40                  45

Ser Leu Gln Thr Gly Phe Leu Ala Ser Leu Phe Tyr Val Lys Asn Phe
    50                  55                  60

Asn Ser Ser Gly Cys Pro Glu Arg Met Ser Ser Cys Arg Glu Leu Asp
65                  70                  75                  80

Asp Phe Arg Ile Gly Trp Gly Thr Leu Glu Tyr Glu Thr Asn Ala Thr
                85                  90                  95

Asn Asp Glu Asp Met Arg Pro Tyr Cys Trp His Tyr Pro Pro Arg Pro
            100                 105                 110

Cys Gly Ile Val Ser Ala Arg Thr Val Cys Gly Pro Val Tyr Cys Phe
        115                 120                 125

Thr Pro Ser Pro Val Val Val Gly Thr Thr Asp Arg Gln Gly Val Pro
    130                 135                 140

Thr Tyr Ser Trp Gly Glu Asn Glu Thr Asp Val Phe Leu Leu Asn Ser
145                 150                 155                 160

Thr Arg Pro Pro Gln Gly Ala Trp Phe Gly Cys Thr Trp Met Asn Gly
                165                 170                 175

Thr Gly Phe Thr Lys Thr Cys Gly Ala Pro Pro Cys Arg Ile Arg Lys
            180                 185                 190

Asp His Asn Ser Thr Leu Asp Leu Leu Cys Pro Thr Asp Cys Phe Arg
        195                 200                 205

Lys His Pro Asp Thr Thr Tyr Leu Lys Cys Gly Ala Gly Pro Trp Leu
    210                 215                 220

Thr Pro Arg Cys Leu Val Asp Tyr Pro Tyr Arg Leu Trp His Tyr Pro
225                 230                 235                 240

Cys Thr Val Asn Phe Thr Ile Phe Lys Val Arg Met Tyr Val Gly Gly
                245                 250                 255

Val Glu His Arg Leu Asn Ala Ala Cys Asn Phe Thr Arg Gly Asp Arg
            260                 265                 270

Cys Arg Leu Glu Asp Arg Asp Arg Gly Gln Gln Ser Pro Leu Leu His
        275                 280                 285

Ser Thr Thr Glu Trp Ala Val Leu Pro Cys Ser Phe Ser Asp Leu Pro
    290                 295                 300

Ala Leu Ser Thr Gly Leu Leu His Leu His Gln Asn Ile Val Asp Val
305                 310                 315                 320

Gln Tyr Leu Tyr Gly Leu Thr Pro Ala Ile Thr Arg Tyr Ile Val Lys
                325                 330                 335

<210>  10
<211>  30
<212>  PRT
<213>  Hepatitis C virus

<400>  10

Asn Ile Gln Leu Ile Asn Thr Asn Gly Ser Trp His Ile Asn Ser Thr
1               5                   10                  15

Ala Leu Asn Cys Asn Glu Ser Leu Asn Thr Gly Trp Leu Ala
            20                  25                  30

<210>  11
<211>  30
<212>  PRT
<213>  Hepatitis C virus

<400>  11

Asn Ile Gln Leu Ile Asn Ser Asn Gly Ser Trp His Ile Asn Arg Thr
1               5                   10                  15

Ala Leu Asn Cys Asn Asp Ser Leu Thr Thr Gly Trp Ile Ala
            20                  25                  30

<210>  12
<211>  30
<212>  PRT
<213>  Hepatitis C virus

<400>  12

Lys Ile Gln Leu Val Asn Thr Asn Gly Ser Trp His Ile Asn Arg Thr
1               5                   10                  15

Ala Leu Asn Cys Asn Asp Ser Leu Asn Thr Gly Phe Leu Ala
            20                  25                  30

<210>  13
<211>  30
<212>  PRT
<213>  Hepatitis C virus

<400>  13

Lys Ile Gln Leu Val Asn Ser Asn Gly Ser Trp His Ile Asn Arg Thr
1               5                   10                  15

Ala Leu Asn Cys Asn Asp Ser Leu Asn Thr Gly Phe Leu Ala
            20                  25                  30

<210>  14
<211>  30
<212>  PRT
<213>  Hepatitis C virus

<400>  14

Asn Ile Gln Leu Ile Asn Thr Asn Gly Ser Trp His Ile Asn Arg Thr
1               5                   10                  15

Ala Leu Asn Cys Asn Asp Ser Leu Gln Thr Gly Phe Ile Ala
            20                  25                  30

<210>  15

<211>  30
<212>  PRT
<213>  Hepatitis C virus

<400>  15

Lys Ile Ser Leu Ile Asn Thr Asn Gly Ser Trp His Ile Asn Arg Thr
1               5                   10                  15

Ala Leu Asn Cys Asn Asp Ser Leu Gln Thr Gly Phe Leu Ala
            20                  25                  30

<210>  16
<211>  30
<212>  PRT
<213>  Hepatitis C virus

<400>  16

Arg Leu Glu Leu Ile Asn Thr Asn Gly Ser Trp His Ile Asn Arg Thr
1               5                   10                  15

Ala Leu Asn Cys Asn Glu Ser Ile Asn Thr Gly Phe Leu Ala
            20                  25                  30

<210>  17
<211>  30
<212>  PRT
<213>  Hepatitis C virus

<400>  17

Asn Val Gln Leu Ile Asn Ser Asn Gly Ser Trp His Ile Asn Arg Thr
1               5                   10                  15

Ala Leu Asn Cys Asn Asp Ser Leu Asn Thr Gly Phe Leu Thr
            20                  25                  30

<210>  18
<211>  30
<212>  PRT
<213>  Hepatitis C virus

<400>  18

Asn Leu Gln Leu Val Asn Thr Asn Gly Ser Trp His Ile Asn Arg Thr
1               5                   10                  15

Ala Leu Asn Cys Gln Asp Ser Leu Gln Thr Gly Phe Ile Ala
            20                  25                  30

<210>  19
<211>  30
<212>  PRT
<213>  Hepatitis C virus

<400>  19

Lys Leu Gln Leu Ile Asn Thr Asn Gly Ser Trp His Ile Asn Arg Thr
1               5                   10                  15

Ala Leu Asn Cys Asn Asp Ser Leu Gln Thr Gly Phe Ile Ala
            20                  25                  30

<210>  20
<211>  30
<212>  PRT
<213>  Hepatitis C virus

<400>  20

Thr Thr Asp Arg Ser Gly Ala Pro Thr Tyr Ser Trp Gly Ala Asn Asp
1               5                   10                  15

Thr Asp Val Phe Val Leu Asn Asn Thr Arg Pro Pro Leu Gly
            20                  25                  30

<210>  21
<211>  30
<212>  PRT
<213>  Hepatitis C virus

<400>  21

Thr Thr Asp Lys Ser Gly Ala Pro Thr Tyr Arg Trp Gly Glu Asn Glu
1               5                   10                  15

Thr Asp Val Phe Val Leu Asn Asn Thr Arg Pro Pro Leu Gly
            20                  25                  30

<210>  22
<211>  30
<212>  PRT
<213>  Hepatitis C virus

<400>  22

Thr Thr Asp Arg Phe Gly Ala Pro Thr Tyr Thr Trp Gly Glu Asn Glu
1               5                   10                  15

Thr Asp Val Leu Ile Leu Asn Asn Thr Arg Pro Pro Gln Gly
            20                  25                  30

<210>  23
<211>  30
<212>  PRT
<213>  Hepatitis C virus

<400>  23

Thr Thr Asp Arg Ser Gly Val Pro Thr Tyr Ser Trp Gly Glu Asn Glu
1               5                   10                  15

Thr Asp Val Leu Leu Leu Asn Asn Thr Arg Pro Pro Gln Gly
            20                  25                  30

<210>  24
<211>  30
<212>  PRT
<213>  Hepatitis C virus

<400>  24

Thr Thr Asn Lys Leu Gly Val Pro Thr Tyr Thr Trp Gly Glu Asn Glu
1               5                   10                  15

Thr Asp Val Phe Leu Leu Asn Ser Thr Arg Pro Pro Arg Gly
            20                  25                  30

<210>  25
<211>  30
<212>  PRT
<213>  Hepatitis C virus

<400>  25

Thr Thr Asp Glu Gln Gly Leu Pro Thr Tyr Ser Trp Gly Glu Asn Glu
1               5                   10                  15

Thr Asp Val Phe Leu Leu Asn Ser Thr Arg Pro Pro Gln Gly
            20                  25                  30

<210>  26
<211>  30
<212>  PRT
<213>  Hepatitis C virus

<400>  26

Thr Thr Asp Pro Lys Gly Ala Pro Thr Tyr Asn Trp Gly Glu Asn Glu
1               5                   10                  15

Thr Asp Val Phe Leu Leu Lys Ser Leu Arg Pro Pro Ser Gly
            20                  25                  30

<210>  27
<211>  30
<212>  PRT
<213>  Hepatitis C virus

<400>  27

Thr Thr Asp Arg Phe Gly Val Pro Thr Tyr Thr Trp Gly Asp Asn Glu
1               5                   10                  15

Ser Asp Val Phe Leu Leu Asn Ala Thr Arg Pro Pro Arg Gly
            20                  25                  30

<210>  28

<211>  30
<212>  PRT
<213>  Hepatitis C virus

<400>  28

Thr Thr Asp Arg Arg Gly Tyr Pro Thr Tyr Asn Trp Gly Ser Asn Glu
1               5                   10                  15

Thr Asp Val Ile Leu Leu Asn Ser Thr Arg Pro Pro Ala Gly
            20                  25                  30

<210>  29
<211>  30
<212>  PRT
<213>  Hepatitis C virus

<400>  29

Thr Thr Asp Arg Arg Gly Asn Pro Thr Tyr Thr Trp Gly Glu Asn Glu
1               5                   10                  15

Thr Asp Val Phe Met Leu Glu Ser Leu Arg Pro Pro Thr Gly
            20                  25                  30

<210>  30
<211>  308
<212>  PRT
<213>  Hepatitis C virus

<400>  30

Ser Leu Ile Asn Thr Asn Gly Ser Trp His Ile Asn Arg Thr Ala Leu
1               5                   10                  15

Asn Cys Asn Asp Ser Leu Gln Thr Gly Phe Leu Ala Ser Leu Phe Tyr
            20                  25                  30

Val Lys Asn Phe Asn Ser Ser Gly Cys Pro Glu Arg Met Ser Ser Cys
        35                  40                  45

Arg Glu Leu Asp Asp Phe Arg Ile Gly Trp Gly Thr Leu Glu Tyr Glu
    50                  55                  60

Thr Asn Ala Thr Asn Asp Glu Asp Met Arg Pro Tyr Cys Trp His Tyr
65                  70                  75                  80

Pro Pro Arg Pro Cys Gly Ile Val Ser Ala Arg Thr Val Cys Gly Pro
                85                  90                  95

Val Tyr Cys Phe Thr Pro Ser Pro Val Val Val Gly Thr Thr Asp Arg
            100                 105                 110

Gln Gly Val Pro Thr Tyr Ser Trp Gly Glu Asn Glu Thr Asp Val Phe

115                 120                 125

Leu Leu Asn Ser Thr Arg Pro Pro Gln Gly Ala Trp Phe Gly Cys Thr
    130                 135                 140

Trp Met Asn Gly Thr Gly Phe Thr Lys Thr Cys Gly Ala Pro Pro Cys
145                 150                 155                 160

Arg Ile Arg Lys Asp His Asn Ser Thr Leu Asp Leu Leu Cys Pro Thr
                165                 170                 175

Asp Cys Phe Arg Lys His Pro Asp Thr Thr Tyr Leu Lys Cys Gly Ala
            180                 185                 190

Gly Pro Trp Leu Thr Pro Arg Cys Leu Val Asp Tyr Pro Tyr Arg Leu
        195                 200                 205

Trp His Tyr Pro Cys Thr Val Asn Phe Thr Ile Phe Lys Val Arg Met
    210                 215                 220

Tyr Val Gly Gly Val Glu His Arg Leu Asn Ala Ala Cys Asn Phe Thr
225                 230                 235                 240

Arg Gly Asp Arg Cys Arg Leu Glu Asp Arg Asp Arg Gly Gln Gln Ser
                245                 250                 255

Pro Leu Leu His Ser Thr Thr Glu Trp Ala Val Leu Pro Cys Ser Phe
            260                 265                 270

Ser Asp Leu Pro Ala Leu Ser Thr Gly Leu Leu His Leu His Gln Asn
        275                 280                 285

Ile Val Asp Val Gln Tyr Leu Tyr Gly Leu Thr Pro Ala Ile Thr Arg
    290                 295                 300

Tyr Ile Val Lys
305

<210>  31
<211>  8
<212>  PRT
<213>  Lama pacos

<400>  31

Gly Phe Thr Asp Asp Tyr Tyr Ala
1               5

<210>  32
<211>  8
<212>  PRT

<213>  Lama pacos

<400>  32

Ile Thr Asn Phe Asp Gly Gly Thr
1               5

<210>  33
<211>  20
<212>  PRT
<213>  Lama pacos

<400>  33

Ala Ala Asp Lys Gly Leu Cys Ser Trp Leu Arg Ala Gly Gly Lys Val
1               5                   10                  15

Thr Phe Gly Ser
            20

<210>  34
<211>  9
<212>  PRT
<213>  Lama pacos

<400>  34

Gly Phe Ser Leu Val Asn Leu Asp Thr
1               5

<210>  35
<211>  8
<212>  PRT
<213>  Lama pacos

<400>  35

Val Ser Glu Ser Asp Gly Lys Glu
1               5

<210>  36
<211>  22
<212>  PRT
<213>  Lama pacos

<400>  36

Val Ala Asp Ser Phe Ala Gln Cys Gln Pro Pro Tyr Ser Gly Trp Asn
1               5                   10                  15

Glu Asp Glu Tyr Asp Tyr
            20